# EUROPEAN PATENT APPLICATION

(11) **EP 4 036 020 A1**
(43) Date of publication of application: **03.08.2022**
(21) Application number: 22153904.2
(22) Date of filing: 28.01.2022
(51) Int. Cl.: B65B 7/16, G01N 35/04

(54) **SPECIMEN PROCESSING METHOD, SPECIMEN PROCESSING SYSTEM, AND SEALING DEVICE**

(30) Priority: 28.01.2021 JP 2021012457
(71) Applicant: SYSMEX CORPORATION, Kobe-shi Hyogo 651-0073 (JP)
(72) Inventor: Yura, Miho, Kobe-shi, Hyogo, 651-0073 (JP); Senda, Go, Kobe-shi, Hyogo, 651-0073 (JP); Nakamichi, Daisuke, Kobe-shi, Hyogo, 651-0073 (JP); Yanagida, Takaichi, Kobe-shi, Hyogo, 651-0073 (JP); Liu, Yanyan, Kobe-shi, Hyogo, 651-0073 (JP); Shimizu, Masato, Kobe-shi, Hyogo, 651-0073 (JP)
(74) Representative: Hoffmann Eitle

(57) **Abstract**

Disclosed is a specimen processing method of processing, by using a specimen processing device, a urine specimen contained in a specimen container having an opening. The method includes: sealing, with a seal, the opening of the specimen container containing the urine specimen; aspirating the urine specimen in the specimen container by using an aspiration tube, of the specimen processing device, which has penetrated through the seal; and processing the aspirated urine specimen.

## Description

### CROSS REFERENCE TO RELATED APPLICATIONS

This application claims priority from prior Japanese Patent Application No. 2021-012457, filed on January 28, 2021, entitled "SPECIMEN PROCESSING METHOD, SPECIMEN PROCESSING SYSTEM, AND SEALING DEVICE", the entire content of which is incorporated herein by reference.

### FIELD OF THE INVENTION

The present invention relates to a specimen processing method, a specimen processing system, and a sealing device used for measuring a urine specimen.

### BACKGROUND

Japanese Laid-Open Patent Publication No. 2017-44631 discloses a urine analyzer for analyzing a urine specimen. When a urine specimen is analyzed, a urine specimen of a subject is collected in a urine collection cup, a fixed quantity of the urine specimen is taken into a specimen container from the urine collection cup, and the specimen container is set in a urine analyzer. The urine analyzer aspirates the urine specimen from an upper opening of the specimen container by using an aspiration tube, and analyzes the urine specimen.

### SUMMARY OF THE INVENTION

Odor of the urine specimen contained in the specimen container set in the urine analyzer is released from the container during measurement, or odor of the urine specimen preserved in the specimen container for a retest leaks through the container during preservation. Such odor makes a user feel unpleasant.

One aspect of the present invention has an object to reduce the unpleasant feeling of the user due to odor of a urine specimen contained in a specimen container.

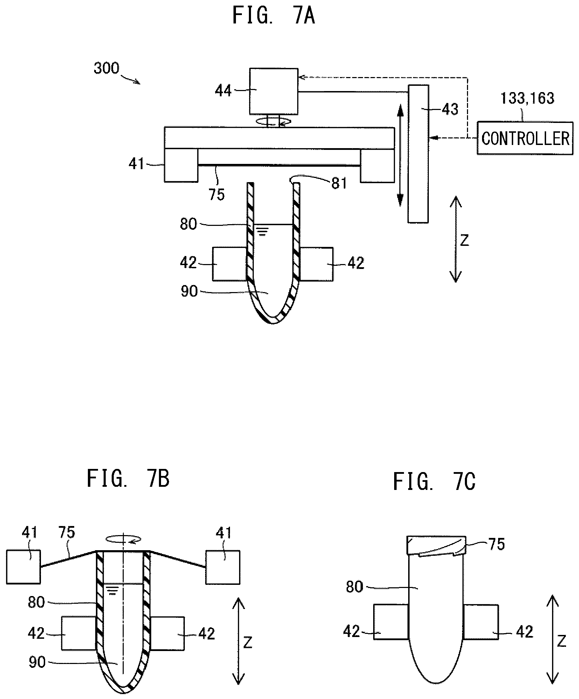

In order to achieve the above object, a specimen processing method according to a first aspect of the present invention is, as shown in FIG. 7, a method of processing, by using a specimen processing device, a urine specimen (90) contained in a specimen container (80) having an opening (81). The method includes: sealing, with a seal (75), the opening (81) of the specimen container (80) containing the urine specimen (90); aspirating the urine specimen (90) in the specimen container (80) by using an aspiration tube (32), of the specimen processing device, which has penetrated through the seal (75) sealing the specimen container (80); and processing the aspirated urine specimen (90).

In the specimen processing method according to the first aspect, aspiration of the urine specimen (90) can be performed with the opening (81) of the specimen container (80) being sealed with the seal (75). Therefore, release of odor can be reduced when the urine specimen (90) is aspirated in the specimen processing. After the aspiration of the urine specimen (90), a hole is formed in the seal (75). However, since the hole formed in the seal (75) is sufficiently smaller than the opening (81) of the specimen container (80), release of odor can be effectively reduced as compared to the case where the opening (81) is fully opened.

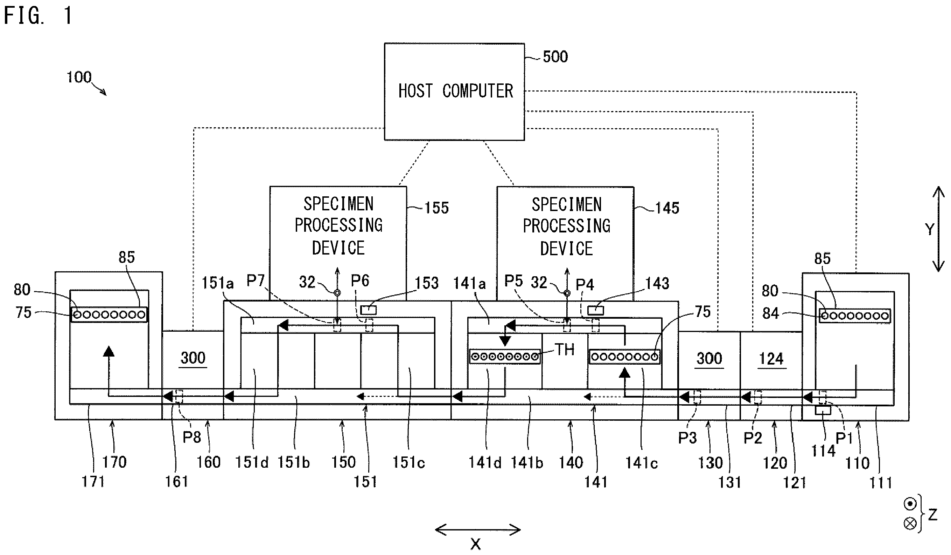

A specimen processing system (100) according to a second aspect of the present invention, as shown in FIG. 1, includes: a sealing device (300) configured to seal, with a seal (75), an opening of a specimen container (80) containing a urine specimen; a transport device (111, 121, 131, 141, 151, 161, 171) configured to transport the sealed specimen container (80); and a specimen processing device (145, 155) including an aspiration tube (32) configured to penetrate through the seal (75) and aspirate the urine specimen from the specimen container (80), the specimen processing device being configured to process the aspirated urine specimen.

In the specimen processing system (100) according to the second aspect, aspiration of the urine specimen can be performed with the opening of the specimen container (80) being sealed with the seal (75), as in the specimen processing method according to the first aspect. Therefore, release of odor can be reduced when the urine specimen is aspirated during the specimen processing. Also, after the aspiration of the urine specimen, release of odor can be effectively reduced as compared to the case where the opening is fully opened.

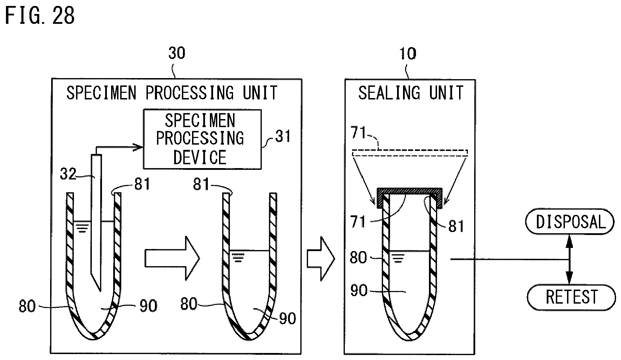

A specimen processing method according to a third aspect of the present invention is, as shown in FIG. 28, a method of processing, by using a specimen processing device (31), a urine specimen (90) contained in a specimen container (80) having an opening (81). The method includes: aspirating the urine specimen (90) from the specimen container (80) containing the urine specimen (90), by using an aspiration tube (32) of the specimen processing device (31); processing the aspirated urine specimen (90); and sealing, with a seal (71), the opening (81) of the specimen container (80) from which the urine specimen (90) has been aspirated.

In the specimen processing method according to the third aspect, since the opening (81) is sealed with the seal (71) after the urine specimen (90) has been aspirated, release of odor from the specimen container (80) can be reduced while the urine specimen (90) is preserved for a retest or the like. When the specimen container (80) sealed with the seal (71) is again subjected to specimen processing for the retest or the like, the aspiration tube (32) can penetrate through the seal (71) and aspirate the urine specimen, which makes an uncapping operation unnecessary. Therefore, complication of uncapping and capping operations can be reduced. Moreover, even when an uncapping operation is performed on a specimen container (80) sealed with a cap, the removed cap need not be stored for recapping, thereby reducing complication of uncapping and capping operations.

A sealing device (300) according to a fourth aspect of the present invention is, as shown in FIG. 7, a sealing device (300) configured to seal an opening (81) of a specimen container (80) having the opening (81). The sealing device (300) includes: a film holder (41) configured to hold a film (75); a container holder (42) configured to hold the specimen container (80) in which a urine specimen (90) is contained; an up-down drive part (43) configured to drive at least one of the film holder (41) and the container holder (42) in an up-down direction; a rotation drive part (44) configured to drive at least one of the film holder (41) and the container holder (42) so as to rotate around a center axis of the specimen container (80); and a controller (133, 163) configured to perform control of winding the film (75) around the specimen container (80) by operating the rotation drive part (44) in a state where the film (75) held by the film holder (41) is, by the up-down drive part (43), pressed against an opening edge of the specimen container (80) held by the container holder (42).

Since the sealing device (300) according to the fourth aspect of the present invention seals the opening (81) of the specimen container (80) with the film (75), specimen aspiration can be performed by the aspiration tube (32) penetrating through the film (75) while the opening (81) is sealed with the film (75). Therefore, release of odor can be reduced when the urine specimen is aspirated in the specimen processing. Although a hole is formed in a portion of the film (75) after the specimen aspiration, release of odor can be effectively reduced as compared to the case where the opening (81) is fully opened.

A specimen processing method according to a fifth aspect of the present invention is a method of processing, by a specimen processing device, a urine specimen contained in a specimen container (80) having an opening. The method includes: sealing, with a seal, an opening of the specimen container (80) containing the urine specimen; forming, by a puncture member of the specimen processing device, a through-hole in the seal with which the specimen container (80) is sealed; aspirating the urine specimen in the specimen container (80) through the through-hole by using an aspiration tube (32); and processing the aspirated urine specimen.

In the specimen processing method according to the fifth aspect of the present invention, aspiration of the urine specimen can be performed through the through-hole, with the opening of the specimen container (80) being sealed with the seal. Therefore, release of odor can be reduced when the urine specimen is aspirated in the specimen processing. After the aspiration of the urine specimen, the through-hole is formed in a portion of the seal. However, since the through-hole formed in the seal is sufficiently smaller than the opening of the specimen container (80), release of odor can be effectively reduced as compared to the case where the opening is fully opened.

According to the present invention, it is possible to reduce the unpleasant feeling of the user due to odor of a urine specimen contained in a specimen container.

### BRIEF DESCRIPTION OF THE DRAWINGS

FIG. 1 is a schematic diagram showing a configuration example of a specimen processing system;
FIG. 2 is a block diagram showing a major configuration of the specimen processing system shown in FIG. 1;
FIG. 3 is a block diagram showing a configuration example of an uncapping device;
FIG. 4 is a diagram for describing a structure of the uncapping device shown in FIG. 3;
FIG. 5A shows a cap gripped by a hand member;
FIG. 5B shows a configuration example of a grip surface of the hand member;
FIG. 6 is a flowchart showing a flow of an uncapping process by an uncapping unit;
FIG. 7A is a diagram for describing a sealing device;
FIG. 7B is a diagram for describing a sealing process;
FIG. 7C is a schematic diagram showing a sealed specimen container;
FIG. 8 is a block diagram showing a configuration example of the sealing device;
FIG. 9 is a perspective view schematically showing a state where a film holder of the sealing device shown in FIG. 8 is located at a film disposal position;
FIG. 10 is a perspective view schematically showing a state where the film holder of the sealing device shown in FIG. 8 is located at a film holding position;
FIG. 11 is a perspective view schematically showing a state where the film holder of the sealing device shown in FIG. 8 is located at a position above a sealing position;
FIGS. 12A to 12D are diagrams for describing an operation of a film piece supply mechanism;
FIGS. 13A to 13E show a flow of a sealing process by the sealing device;
FIG. 14 is a schematic diagram showing a sealing mechanism and a film piece disposal mechanism;
FIG. 15 is an enlarged view showing a sealed portion of a specimen container;
FIG. 16 is a flowchart showing a flow of a sealing process by the sealing device;
FIG. 17 is a schematic diagram showing transport devices of a sealing unit and a specimen processing unit;
FIG. 18 is a schematic diagram showing a measurement part of a urine qualitative test device;
FIG. 19 is a schematic diagram showing a measurement part of a urinary sediment test device;
FIG. 20 is a block diagram for describing a computer implementing a controller;
FIG. 21 is a flowchart showing a processing operation flow of the specimen processing system;
FIG. 22 is a diagram showing a configuration example in which a first loading unit and a second loading unit are disposed;
FIG. 23 is a diagram showing a first configuration example in which a third loading unit and a fourth loading unit are disposed;
FIG. 24 is a diagram showing a second configuration example in which a third loading unit and a fourth loading unit are disposed;
FIG. 25 is a diagram showing a configuration example in which each of a plurality of specimen processing units is provided with a second sealing unit;
FIG. 26 is a diagram showing a configuration example in which an uncapping unit and a second sealing unit are not disposed;
FIG. 27 is a diagram showing a configuration example in which a sealing unit is disposed not on an upstream side but on a downstream side of a specimen processing device;
FIG. 28 is a diagram for describing a third specimen processing method;
FIGS. 29A to 29H are schematic diagrams for describing a flow of a second sealing method;
FIG. 30 is an enlarged view of a sealed portion of a specimen container by the second sealing method;
FIG. 31 is a diagram showing a sealing method in which a film and a specimen container are not relatively rotated;
FIG. 32 is a diagram showing a configuration example in which a film is held by means of pressure;
FIG. 33 is a diagram showing an example in which a film is pushed into an opening of a specimen container by means of pressure;
FIG. 34 is a diagram showing a configuration example in which a plurality of specimen containers are collectively sealed;
FIGS. 35A and 35B are diagrams showing control for upward movement of a specimen container in a first example in which a detector for a specimen container is disposed; and
FIGS. 36A and 36B are diagrams showing control for upward movement of a specimen container in a second example in which a detector for a specimen container is disposed.

### DETAILED DESCRIPTION OF THE PREFERRED EMBODIMENTS

Hereinafter, embodiments will be described with reference to the drawings.

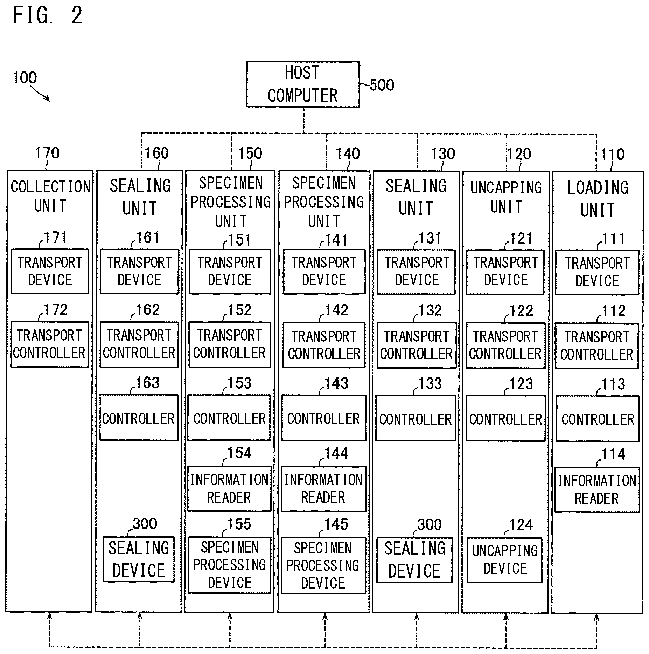

With reference to FIG. 1 and FIG. 2, an overall configuration of a specimen processing system 100 will be described.

The specimen processing system 100 shown in FIG. 1 includes a loading unit 110, an uncapping unit 120, a sealing unit 130, a specimen processing unit 140, a specimen processing unit 150, a second sealing unit 160, and a collection unit 170. The loading unit 110, the uncapping unit 120, the sealing unit 130, the specimen processing unit 140, the specimen processing unit 150, the sealing unit 160, and the collection unit 170 include transport devices 111, 121, 131, 141, 151, 161, and 171, respectively. In the following description, a first seal attached to a specimen container 80 in advance is a cap 84, and a film 75 is used as a seal and a second seal. In each figure, a transport direction in which specimen containers 80 are transported by the transport device is an X direction. A direction orthogonal to the X direction in a horizontal plane is a Y direction. An up-down direction orthogonal to the X direction and the Y direction is a Z direction.

In the example of FIG. 1, the loading unit 110, the uncapping unit 120, the sealing unit 130, the specimen processing unit 140, the specimen processing unit 150, the sealing unit 160, and the collection unit 170 are arranged in this order from the upstream side to the downstream side in the transport direction (X direction) of the specimen containers 80. The respective units are mutually connected such that the specimen containers 80 can be transported between adjacent units by the plurality of transport devices.

Each unit will be described with reference to FIG. 1 and FIG. 2.

The loading unit 110 has a function of providing a place for the specimen containers 80 to be loaded into the specimen processing system 100, and sending each loaded specimen container 80 toward the downstream side. Each specimen container 80 contains a urine specimen 90 (see FIG. 7). The specimen containers 80, being set in a specimen rack 85, are set in the loading unit 110 by a user. The specimen rack 85 is a rack capable of holding a plurality of specimen containers 80 such that the specimen containers 80 stand straight with openings 81 thereof facing upward.

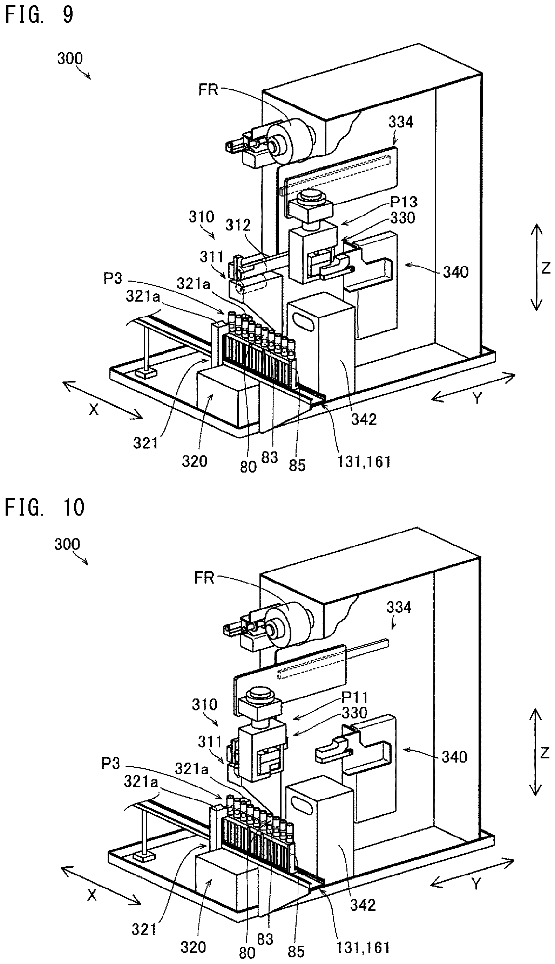

The loading unit 110 (see FIG. 2) includes a transport device 111, a transport controller 112, a controller 113, and an information reader 114. The transport device 111 is configured to transport a specimen rack 85 placed in the loading unit 110 to the uncapping unit 120. The information reader 114 is configured to read identification information of each specimen container 80 held in the specimen rack 85 transported by the transport device 111. The identification information includes a unique specimen ID identifying the urine specimen 90 contained in the specimen container 80. For example, identification information is printed on an identification label 83 (see FIG. 9) in a form such as a one-dimensional code or a two-dimensional code, and the identification label 83 is pasted to the side surface of each specimen container 80. The information reader 114 is implemented by a code reader or a camera, and optically reads identification information.

The controller 113 described later in detail is configured to be communicable with a host computer 500. The controller 113 can acquire specimen information registered in the host computer 500, by using identification information acquired by the information reader 114. The specimen information includes various kinds of information, related to specimen processing, such as items to be measured by the specimen processing unit 140 and the specimen processing unit 150, presence/absence of a cap, necessity of sealing with a seal, and the like. The transport controller 112 includes a processor and a memory. The transport controller 112 controls the transport device 111 so as to transport the specimen rack 85 to the uncapping unit 120. The transport controller 112 performs mutual communication with transport controllers of the other units in the specimen processing system 100, and controls a transport start timing for the specimen rack 85 in accordance with the states of the transport device in the respective units.

The uncapping unit 120 is configured to receive the specimen rack 85 from the loading unit 110, and perform an uncapping process on each specimen container 80 set in the specimen rack 85. That is, the uncapping unit 120 is configured to remove a cap 84 from a specimen container 80 having an opening 81 to which the cap 84 is attached. The uncapping unit 120 is configured to send, to the sealing unit 130, the specimen container 80 from which the cap 84 has been removed through the uncapping process.

The uncapping unit 120 (see FIG. 2) includes a transport device 121, a transport controller 122, a controller 123, and an uncapping device 124. The transport device 121 is configured to transport the specimen rack 85 between the loading unit 110 and the sealing unit 130. The transport device 121 transports the specimen containers 80 held in the specimen rack 85 to an uncapping position P2 (see FIG. 1) in the uncapping device 124. The uncapping position P2 is present on a transport path between the loading unit 110 and the sealing unit 130. The uncapping device 124 is configured to perform an uncapping process of removing a cap 84 from a specimen container 80 transported to the uncapping position P2. The uncapping device 124 will be described later in detail. The controller 123 controls the operation of the uncapping device 124 so as to uncap the specimen containers 80 transported to the uncapping position P2 one by one. The transport controller 122 includes a processor and a memory. The transport controller 122 controls the transport device 121 so as to receive the specimen rack 85 from the loading unit 110 and transport the specimen containers 80 held in the specimen rack 85 one by one to the uncapping position P2 in the uncapping device 124. The transport controller 122 controls the transport device 121 so as to transport, to the sealing unit 130, the specimen rack 85 holding the specimen containers 80 having been uncapped.

The sealing unit 130 is configured to seal, with a film 75, a specimen container 80 from which a cap 84 has been removed. Thus, the specimen processing method of the present embodiment further includes a step of removing a cap 84 from a specimen container 80 having an opening 81 to which the cap 84 is attached, and a step of sealing, with a film 75, the specimen container 80 from which the cap 84 has been removed. Thus, even when the cap 84 has been attached to the specimen container 80 in advance, the cap 84 can be replaced with the film 75 through which an aspiration tube 32 can penetrate. Therefore, the user need not remove a cap 84 from each specimen container 80 before loading the specimen containers 80 into the specimen processing unit 140. Moreover, a time period during which the opening 81 of each specimen container 80 is opened can be limited to a time period from when the cap 84 is removed to when the opening 81 is sealed with the film 75. Therefore, it is possible to effectively reduce release of odor of the urine specimen 90 to the outside of the specimen container 80.

The sealing unit 130 (see FIG. 2) includes a transport device 131, a transport controller 132, a controller 133, and a sealing device 300. The transport device 131 is configured to transport the specimen rack 85 between the uncapping unit 120 and the specimen processing unit 140. When the specimen rack 85 is received from the uncapping unit 120 into the sealing unit 130, the transport device 131 transports the specimen containers 80 held in the specimen rack 85 to a sealing position P3 (see FIG. 1) in the sealing device 300. The sealing position P3 is present on a transport path between the uncapping unit 120 and the specimen processing unit 140. The sealing device 300 is configured to perform a sealing process of sealing, with the film 75, the opening 81 of each specimen container 80 transported to the sealing position P3. The sealing device 300 will be described later in detail. The controller 133 controls the operation of the sealing device 300 so as to seal the specimen containers 80 transported to the sealing position P3 one by one. The transport controller 132 includes a processor and a memory. The transport controller 132 controls the transport device 131 so as to receive the specimen rack 85 from the uncapping unit 120, and transport the specimen containers 80 held in the specimen rack 85 one by one to the sealing position P3 in the sealing device 300. The transport controller 132 controls the transport device 131 so as to transport, to the specimen processing unit 140, the specimen rack 85 holding the specimen containers 80 having been subjected to the sealing process.

The specimen processing unit 140 aspirates, by using the aspiration tube 32, the urine specimen 90 (see FIG. 7) from the specimen container 80 to which the film 75 is attached by the sealing unit 130, and performs measurement on the aspirated urine specimen 90.

The specimen processing unit 140 (see FIG. 2) includes a transport device 141, a transport controller 142, a controller 143, an information reader 144, and a specimen processing device 145. The transport device 141 is configured to transport the specimen rack 85 between the sealing unit 130 and the specimen processing unit 150. The transport device 141 transports the specimen containers 80 held in the specimen rack 85 to a reading position P4 (see FIG. 1) in the information reader 144 and to an aspiration position P5 (see FIG. 1) in the specimen processing device 145. The reading position P4 and the aspiration position P5 are present on a first transport path 141a. The information reader 144 has the same configuration as the information reader 114, and reads identification information of each specimen container 80 transported to the reading position P4. The specimen processing device 145 causes the aspiration tube 32 to penetrate through the film 75 of each specimen container 80 transported to the aspiration position P5, and aspirate the urine specimen 90 from the specimen container 80. The specimen processing device 145 performs specimen processing on the urine specimen 90 aspirated by the aspiration tube 32. After the film 75 has been punctured by the aspiration tube 32, the specimen container 80 is transported with a through-hole TH formed in the film 75 until it is re-sealed by the second sealing unit 160.

The transport device 141 includes a first transport path 141a, a second transport path 141b, a loading/storage part 141c, and a sending/storage part 141d, and is capable of receiving/transporting a specimen rack 85 from/to the second transport path 141b. The first transport path 141a, the second transport path 141b, the loading/storage part 141c, and the sending/storage part 141d form a loop path, so that the transport device 141 can circularly transport the specimen rack 85 for a retest. The second transport path 141b allows the transport device 141 to transport the specimen rack 85 from the sealing unit 130 to the specimen processing unit 150 not through the specimen processing device 145.

The controller 143 acquires measurement order information from the host computer 500 on the basis of identification information read by the information reader 144. On the basis of the measurement order information, the controller 143 controls the operation of the specimen processing device 145 so as to perform or so as not to perform specimen aspiration from each of individual urine specimens 90 (individual specimen containers 80). The controller 143 controls the operation of the specimen processing device 145 so as not to perform specimen aspiration from a specimen container 80 for which specimen processing by the specimen processing unit 140 is not required according to the measurement order information.

The transport controller 142 includes a processor and a memory. The transport controller 142 controls the transport device 141 so as to receive the specimen rack 85 from the sealing unit 130 and transport the specimen containers 80 held in the specimen rack 85 one by one to the reading position P4 and the aspiration position P5. The transport controller 142 controls the transport device 141 so as to transport, to the specimen processing unit 150, the specimen rack 85 holding the specimen containers 80 having been subjected to specimen aspiration.

The specimen processing unit 150 aspirates, by using the aspiration tube 32, the urine specimen 90 (see FIG. 7) from the specimen container 80 received from the transport device 141 of the specimen processing unit 140, and performs measurement on the aspirated urine specimen 90.

The specimen processing unit 150 (see FIG. 2) includes a transport device 151, a transport controller 152, a controller 153, an information reader 154, and a specimen processing device 155. The hardware configurations of the transport device 151, the information reader 154, the controller 153, and the transport controller 152 are similar to those of the specimen processing unit 140.

The transport device 151 is configured to transport the specimen rack 85 between the specimen processing unit 140 and the second sealing unit 160. The transport device 151 transports the specimen containers 80 held in the specimen rack 85 to a reading position P6 (see FIG. 1) in the information reader 154 and to an aspiration position P7 (see FIG. 1) in the specimen processing device 155. The reading position P6 and the aspiration position P7 are present on a first transport path 151a. The specimen processing device 155 causes the aspiration tube 32 to penetrate through the film 75 of each specimen container 80 transported to the aspiration position P7, and aspirate the urine specimen 90 from the specimen container 80. The specimen processing device 155 performs specimen processing on the urine specimen 90 aspirated by the aspiration tube 32.

The transport device 151, similar to the transport device 141, includes the first transport path 151a, a second transport path 151b, a loading/storage part 151c, and a sending/storage part 151d. The second transport path 151b allows the transport device 151 to transport the specimen rack 85 from the specimen processing unit 140 to the collection unit 170 not through the specimen processing device 155. The controller 153 acquires measurement order information from the host computer 500 on the basis of identification information read from the information reader 154. On the basis of the measurement order information, the controller 153 controls the operation of the specimen processing device 155 so as to perform or so as not to perform specimen aspiration from each of the individual urine specimens 90 (individual specimen containers 80). As for a specimen container 80 having been subjected to specimen aspiration in the upstream-side specimen processing unit 140, the film 75 thereof is again punctured by the aspiration tube 32 of the specimen processing device 155.

The transport controller 152 controls the transport device 151 so as to receive the specimen rack 85 from the specimen processing unit 140 and transport the specimen containers 80 held in the specimen rack 85 one by one to the reading position P6 and the aspiration position P7. The transport controller 152 controls the transport device 151 so as to transport, to the second sealing unit 160, the specimen rack 85 holding specimen containers 80 having been subjected to specimen aspiration or specimen containers 80 not to be subjected specimen processing.

The second sealing unit 160 is configured to re-seal, with a film 75, the opening 81 of each specimen container 80 from which the urine specimen 90 has been aspirated. Thus, the specimen processing method of the present embodiment further includes a step of re-sealing, with a film 75, an opening 81 of a specimen container 80 whose film 75 has been punctured by the aspiration tube 32 and from which a urine specimen 90 has been aspirated. The second sealing unit 160 performs the re-sealing process such that the previously attached film 75 is not removed from the specimen container 80 and is covered with the new film 75.

Thus, even when the through-hole TH is formed by the aspiration tube 32 in the film 75 attached to the opening 81 before aspiration, the opening 81 can be re-sealed with the new film 75, whereby release of odor of the urine specimen 90 to the outside of the specimen container 80 can be reduced more effectively. Moreover, since the opening 81 can be re-sealed with the film 75, the specimen container 80 having been subjected to specimen aspiration can be preserved for a retest or the like.

The hardware configuration of the sealing unit 160 is similar to that of the upstream-side sealing unit 130. The transport device 161 is configured to transport the specimen rack 85 between the specimen processing unit 150 and the collection unit 170. Moreover, the transport device 161 transports the specimen containers 80 held in the specimen rack 85 to a sealing position P8 (see FIG. 1) present on a transport path between the specimen processing unit 150 and the collection unit 170. The controller 163 controls the operation of the sealing device 300 so as to seal the specimen containers 80 transported to the sealing position P8 one by one.

The transport controller 162 controls the transport device 161 so as to receive the specimen rack 85 from the specimen processing unit 150 and transport the specimen containers 80 held in the specimen rack 85 one by one to the sealing position P8 in the sealing device 300. The transport controller 162 controls the transport device 161 so as to transport, to the collection unit 170, the specimen rack 85 holding the specimen containers 80 having been subjected to the sealing process.

The collection unit 170 has a function of receiving the specimen containers 80 having been subjected to specimen processing by the specimen processing system 100, and providing a storage place for the received specimen containers 80. The collection unit 170 includes a transport device 171 and a transport controller 172. The transport device 171 is configured to receive the specimen rack 85 from the sealing unit 160, and transport the received specimen rack 85 to the storage position. The transport controller 172 includes a processor and a memory. The transport controller 172 controls a timing to receive the specimen rack 85 by the transport device 171, and controls the transport device 171 so as to transport the received specimen rack 85 to the storage position.

### (Configuration example of uncapping unit)

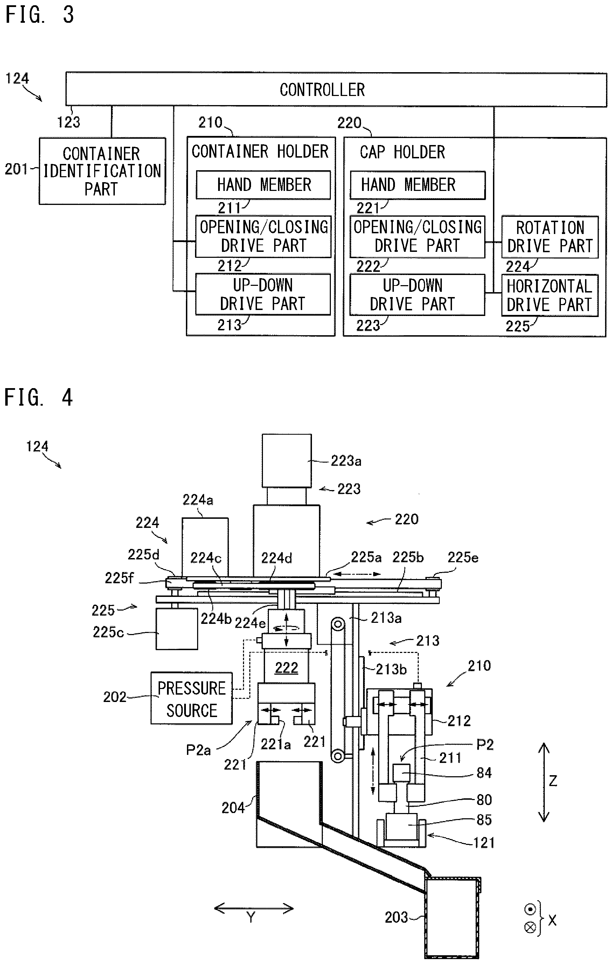

With reference to FIG. 3 to FIG. 5B, the configuration example of the uncapping unit 120 will be described. As shown in FIG. 3, the uncapping device 124 of the uncapping unit 120 is roughly divided by function into a controller 123, a container identification part 201, a container holder 210, and a cap holder 220.

As shown in FIG. 1, the specimen rack 85 set in the loading unit 110 is placed at an outlet of the transport device 111. When a state where the specimen rack 85 can be carried out from an outlet of the loading unit 110 and a state where the specimen rack 85 can be carried into an inlet of the uncapping unit 120 have been established, the specimen rack 85 is transversely carried out from the outlet of the loading unit 110 and is carried into the inlet of the uncapping unit 120.

### (Identification of specimen container)

As shown in FIG. 3, the uncapping device 124 includes a camera as the container identification part 201. The container length, presence/absence of a cap 84, the type of the cap 84 if any, and the like are identified by the container identification part 201 (see FIG. 3) while the specimen rack 85 is intermittently transported in the transverse direction by the transport device 121 as shown in FIG. 1. When the controller 123 (see FIG. 3) has determined that a specimen container 80 is a container to be uncapped, on the basis of the identification result of the container identification part 201, this specimen container 80 is subjected to an uncapping process suitable for the type of the cap 84 thereof at the uncapping position P2. When the controller 123 has determined that a specimen container 80 is not a container to be uncapped, this specimen container 80 is not subjected to an uncapping process.

### (Uncapping process)

When the specimen container 80 to be uncapped is located at the uncapping position P2 (see FIG. 1), the transport controller 122 (see FIG. 2) controls the transport device 121 so as to temporarily stop transport of the specimen rack 85. The controller 123 (see FIG. 3) controls the uncapping device 124 so as to perform the uncapping process on the specimen container 80.

### (Structure of uncapping device)

The container holder 210 includes hand members 211 for holding a specimen container 80, an opening/closing drive part 212 for the hand members 211, and an up-down drive part 213 for the hand members 211. Specifically, a pair of hand members 211 are disposed opposing each other such that a specimen container 80 can be placed therebetween. As shown in FIG. 4, the opening/closing drive part 212 opens and closes the pair of hand members 211 with a pressure supplied from a pressure source 202. The opening/closing drive part 212 causes the pair of hand members 211 to hold a specimen container 80 by moving them in a direction in which they approach each other. The opening/closing drive part 212 causes the pair of hand members 211 to release the specimen container 80 by moving them in a direction in which they move away from each other. The up-down drive part 213 is configured to move the pair of hand members 211 and the opening/closing drive part 212 along a rail 213b extending in the up-down direction (Z direction) in accordance with an operation of a motor 213a.

The cap holder 220 (see FIG. 3) includes hand members 221 for holding a cap 84, an opening/closing drive part 222 for the hand members 221, an up-down drive part 223 for the hand members 221, a rotation drive part 224 for the hand members 221, and a horizontal drive part 225 for the hand members 221.

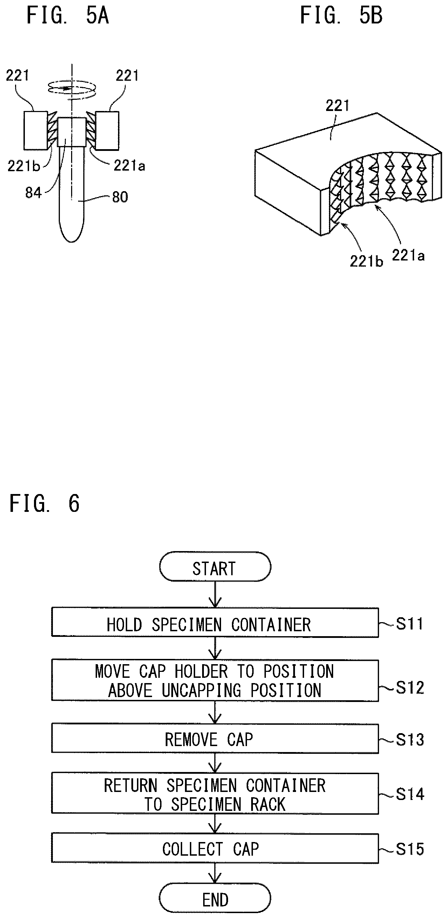

As shown in FIG. 4, a pair of hand members 221 are disposed opposing each other such that a cap 84 of a specimen container 80 can be placed therebetween. On the opposing side surfaces of the pair of hand members 221, grip faces 221a for the cap 84 are formed. As shown in FIG. 5A and FIG. 5B, on each grip face 221a, a plurality of acute projections 221b are formed upward (in a direction of pulling out the cap 84). As the cap 84, a rubber cap, a resin cap, a rubber/resin composite cap (inside: rubber, outside: resin), or the like may be used. The projections 221b ensure high holding force of the cap 84 of any type.

Referring back to FIG. 4, the opening/closing drive part 222 opens and closes the pair of hand members 221 by means of pressure supplied from a pressure source 202. The opening/closing drive part 222 causes the pair of hand members 221 to hold the cap 84 by moving them in a direction in which they approach each other. The opening/closing drive part 212 causes the pair of hand members 221 to release the cap 84 by moving them in a direction in which they move away from each other. The up-down drive part 223 holds the opening/closing drive part 222 via a spline shaft 224e extending in the up-down direction, and moves the pair of hand members 221 and the opening/closing drive part 222 in the up-down direction by changing the delivery amount of the spline shaft 224e in the up-down direction (Z direction) in accordance with an operation of the motor 223a. The rotation drive part 224 causes a motor 224a to rotate a driving pulley 224b attached to an output shaft of the motor 224a, thereby rotating, via a belt 224c, a driven pulley 224d connected to the spline shaft 224e. Thus, the rotation drive part 224 causes the opening/closing drive part 222 and the pair of hand members 221 which are held at a lower end of the spline shaft 224e to rotate around the center axis in the up-down direction.

The horizontal drive part 225 includes a holding member 225a which holds the hand members 221, the opening/closing drive part 222, the up-down drive part 223, and the rotation drive part 224, on a rail 225b, movably in the horizontal direction. The rail 225b extends along the Y direction. The horizontal drive part 225 causes a motor 225c to rotate a driving pulley 225d attached to an output shaft of the motor 225c, thereby rotating a belt 225f between the driving pulley 225d and a driven pulley 225e. The holding member 225a is connected to the belt 225f. Thus, the horizontal drive part 225 horizontally moves the pair of hand members 221 held by the holding member 225a to a position above the specimen container 80 held by the container holder 210 (i.e., a position immediately above the uncapping position P2) and to a stand-by position P2a off from the position above the specimen container 80. A chute part 204 connected to a collection container 203 is disposed beneath the stand-by position P2a.

### <Uncapping process>

The controller 123 (see FIG. 3) controls the uncapping device 124 configured as described above so as to operate as follows, thereby implementing an uncapping process.

In step S11 shown in FIG. 6, the controller 123 controls the opening/closing drive part 212 (pressure source 202) so as to close the pair of hand members 211. A specimen container 80 sent to the uncapping position P2 is held by the pair of hand members 211.

In step S12, the controller 123 controls the horizontal drive part 225 so as to move the pair of hand members 221 to the position above the uncapping position P2.

In step S13, the controller 123 controls the container holder 210 and the cap holder 220 so as to remove a cap 84 from the specimen container 80. Firstly, the controller 123 controls the up-down drive part 213 so as to pull the specimen container 80 held by the hand members 211 upward from the specimen rack 85. Moreover, the controller 123 controls the up-down drive part 223 so as to move the pair of hand members 221 downward such that the cap 84 is placed between the pair of hand members 221.

Next, the controller 123 controls the opening/closing drive part 222 (pressure source 202) so as to close the pair of hand members 221. The cap 84 of the specimen container 80 is held by the pair of hand members 221. Next, the controller 123 controls the up-down drive part 223 and the rotation drive part 224 such that the pair of hand members 221 are moved upward while being rotated (see FIG. 5A). Thus, the cap 84 held by the pair of hand members 221 is removed upward from the specimen container 80.

The controller 123 changes the rotation angle of the hand members 221 on the basis of the identification result of the container identification part 201. According to the type of the cap 84 identified, the rotation angle is set to a predetermined angle, e.g., 450° for a screw-type cap 84, 90° for a push-in type cap 84, or the like. The pair of hand members 221 may be rotated while being closed, may be rotated in a reverse direction while being opened, and may be rotated again while being closed. This control is useful when the angle of rotation of the hand members 221 by the rotation drive part 224 is limited. The cap 84 can be rotated by an arbitrary angle by performing the rotation operation a plural number of times.

Thereafter, in step S14, the controller 123 controls the up-down drive part 213 so as to move the specimen container 80 held by the hand members 211 downward and return the specimen container 80 to the specimen rack 85. Then, the controller 123 controls the opening/closing drive part 212 (pressure source 202) so as to open the pair of hand members 211. Thus, holding of the uncapped specimen container 80 is released, and the specimen container 80 held by the pair of hand members 211 is returned to the specimen rack 85. In this case, the controller 123 may control the amount of the downward movement of the pair of hand members 211 by the up-down drive part 213 so as to adjust the depth at which the specimen container 80 is inserted in the specimen rack 85. This avoids peeling of the identification label 83 (see FIG. 9) pasted to the specimen container 80.

In step S15, the removed cap 84 is collected. The controller 123 controls the horizontal drive part 225 so as to move the pair of hand members 221 to the stand-by position P2a. The controller 123 controls the opening/closing drive part 222 (pressure source 202) so as to open the pair of hand members 221. Then, the cap 84 held by the pair of hand members 221 falls downward from the stand-by position P2a into the chute part 204. The cap 84 is collected into the collection container 203 through the chute part 204. When a predetermined number of caps 84 have been stored in the collection container 203, the collection container 203 is removed by the user, and the caps 84 are discarded, or washed and reused.

### (Configuration example of sealing unit)

With reference to FIG. 7A to FIG. 17, a configuration example of the sealing unit 130 will be described. As described above, the configuration of the sealing unit 160 is similar to that of the sealing unit 130.

### (Seal)

A seal that the sealing unit 130 uses for sealing an opening 81 of a specimen container 80 is obtained by adhering a thin member made of a resin, a metal, or the like, to an edge of the opening 81 and a peripheral wall, near the edge, of the specimen container 80 through bonding, thermal welding, cohesion, or the like.

As described above, in the present embodiment, the seal (second seal) is the film 75. In this specification, "film" means a thin film-like member, and represents a broad concept including "sheet", "membrane", and "foil". As for the film 75 as the seal, a combination of a constituent material and a thicknesses thereof is limited within a range in which the aspiration tube 32 can puncture the film 75.

Therefore, the opening 81 of the specimen container 80 can be easily sealed with the film 75 which can be easily punctured, and release of odor can be effectively reduced by the film 75. Although a rubber cap is conceivable as a seal that can be punctured by the aspiration tube 32, great force is required for puncturing through the rubber cap. The film 75, which is sufficiently thinner than such a rubber cap, is preferable because it allows use of an aspiration mechanism having a simple structure.

Specifically, the film 75 is a stretchable resin film. The stretchable resin film has excellent sealability, and therefore can effectively reduce release of odor of a urine specimen from a specimen container. As the stretchable resin film, a film that is irreversibly stretchable and exhibits self-adhesiveness when being stretched is preferably used.

Thus, the film 75 can be stretched and adhered to the specimen container 80. Therefore, it is possible to realize a sealing structure by which the opening 81 of the specimen container 80 can be easily sealed and the sealing is not easily released.

The stretchable resin film is a plastic paraffin film, for example. The thickness of the resin film is, for example, 1 mm or less, preferably 0.5 mm or less, and more preferably 0.2 mm or less.

### (Sealing device)

Each of the sealing unit 130 and the sealing unit 160 (see FIG. 1) of the specimen processing system 100 of the present embodiment includes a sealing device 300 as shown in FIGS. 7A to 7C. The sealing device 300 is a device for sealing an opening 81 of a specimen container 80 having the opening 81.

A schematic configuration of the sealing device 300 of the present embodiment will be described. As shown in FIG. 7A, the sealing device 300 includes a film holder 41, a container holder 42, an up-down drive part 43, a rotation drive part 44, and a controller (133, 163).

The film holder 41 is configured to hold a film 75 as a seal. The container holder 42 is configured to hold a specimen container 80 containing a urine specimen 90. The up-down drive part 43 is configured to drive the film holder 41 in the up-down direction (Z direction). The rotation drive part 44 is configured to drive and rotate the film holder 41 around the center axis of the specimen container 80. The up-down drive part 43 may drive the container holder 42 in the up-down direction, and the rotation drive part 44 may drive and rotate the container holder 42 around the center axis of the specimen container 80.

The controller (133, 163) is configured to perform control of winding the film 75 held by the film holder 41 around the specimen container 80 by operating the rotation drive part 44 while the film 75 is pressed by the up-down drive part 43 against the opening edge of the specimen container 80 held by the container holder 42.

As shown in FIG. 7B, the film 75 horizontally held by the film holder 41 is pressed from above against the opening edge at the upper end of the specimen container 80. The opening edge of the specimen container 80 is located a little above the position where the film 75 is held, and a tension is applied to the film 75 by the specimen container 80. The film 75 is stretched and protruded upward. Thus, the film 75 covering the opening 81 is pressed against and adhered to the opening edge. With the film 75 being stretched, the film holder 41 and the container holder 42 are relatively rotated around the center axis of the specimen container 80. Thus, the film 75 adhered to the opening edge is wound around the outer peripheral surface of the specimen container 80. As shown in FIG. 7C, the film 75 is adhered to the opening edge of the specimen container 80 and to the outer peripheral surface, of the specimen container 80, extending downward from the opening edge. As a result, the opening 81 of the specimen container 80 is sealed with the film 75.

### (Effect of sealing device)

As described above, the sealing device 300 of the present embodiment is provided with the controller (133, 163) which performs control of winding the film 75 held by the film holder 41 around the specimen container 80 by operating the rotation drive part 44 while the film 75 is pressed against the opening edge of the specimen container 80 held by the container holder 42. Thus, the opening 81 of the specimen container 80 is sealed with the film 75. Therefore, specimen aspiration can be performed by the aspiration tube 32 penetrating through the film 75 while the opening 81 is sealed with the film 75. Therefore, release of odor can be reduced when the urine specimen is aspirated in the specimen processing. Although a hole is formed in a portion of the film 75 after the specimen aspiration, release of odor can be effectively reduced as compared to the case where the opening 81 is fully opened.

In the specimen processing method of the present embodiment, the step of sealing the opening 81 of the specimen container 80 with the seal includes: the step of stretching the film 75 by pressing the opening edge of the specimen container 80 against the film 75; and the step of winding the seal around the specimen container 80 by rotating the stretched film 75 around the center axis of the specimen container 80.

Therefore, sealing can be performed such that the film 75 is wound around the opening edge and the side surface of the specimen container 80 after the opening 81 of the specimen container 80 is sealed with the film 75. As a result, the opening 81 of the specimen container 80 can be firmly sealed, thereby effectively preventing not only leakage of odor but also leakage of the urine specimen 90 when the specimen container 80 is tilted or the urine specimen 90 is agitated.

### (Detailed configuration of sealing device)

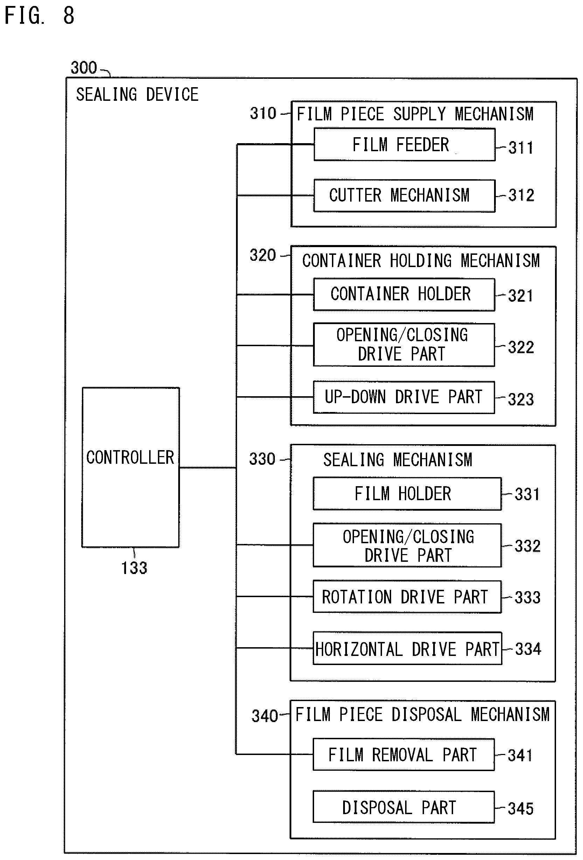

FIG. 8 to FIG. 11 show the configuration of the sealing device 300 in detail. As shown in FIG. 8, the sealing device 300 is roughly divided by function into a controller 133, a film piece supply mechanism 310, a container holding mechanism 320, a sealing mechanism 330, and a film piece disposal mechanism 340. FIG. 8 and FIG. 9 show an example of a configuration in which an up-down drive part 323 of the container holding mechanism 320 drives a container holder 321 in an up-down direction, and a rotation drive part 333 of the sealing mechanism 330 drives and rotates a film holder 331.

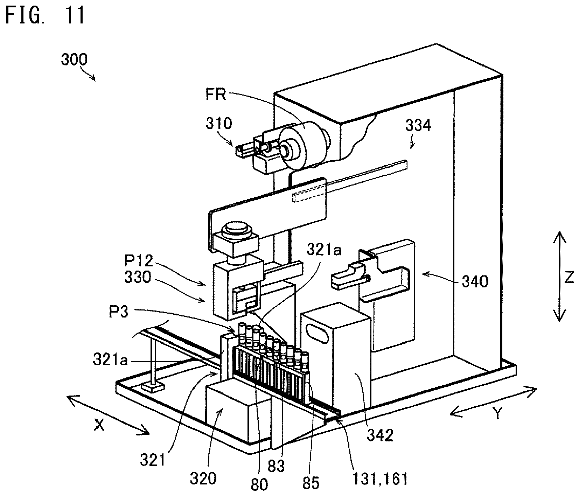

As shown in FIG. 9 to FIG. 11, the sealing mechanism 330 is movable in the Y direction, and is moved to a film holding position P11 (see FIG. 10), a position P12 above the sealing position P3 (see FIG. 11), and a film disposal position P13 (see FIG. 9).

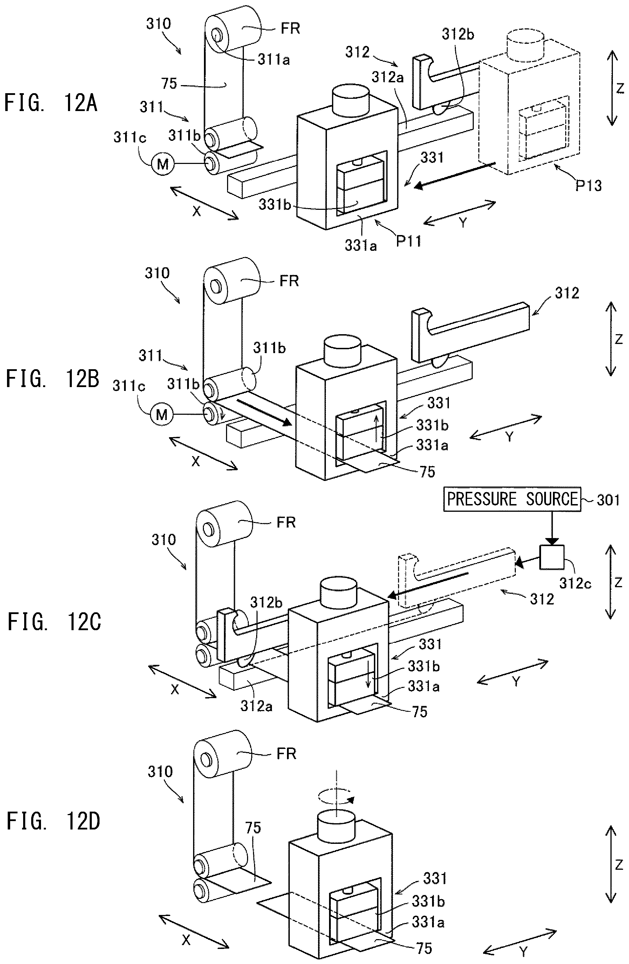

The film piece supply mechanism 310 is configured to hold a film roll FR (i.e., rolled film 75), and feed the film 75 to the film holder 331 disposed at the film holding position P11 (see FIG. 10). As shown in FIG. 12A, the film piece supply mechanism 310 is disposed side by side, in the X direction, with the film holder 331 disposed at the film holding position P11.

The film piece supply mechanism 310 includes a film feeder 311 and a cutter mechanism 312. As shown in FIG. 12A, the film feeder 311 includes: a roll holder 311a which holds the film roll FR of the film 75 rotatably around a winding shaft thereof; a pair of feeding rollers 311b; and a roller drive part 311c. The film 75 pulled out from the film roll FR is set to be sandwiched by the pair of feeding rollers 311b. The pair of feeding rollers 311b has a function such that one roller, i.e., a driving roller, is driven and rotated by the roller drive part 311c while the other roller, i.e., a driven roller, presses the film 75 toward the driving roller side. As shown in FIG. 12B, the roller drive part 311c rotates the driving roller by a predetermined amount, whereby the film 75 is fed out by the predetermined amount in the horizontal direction (X direction) from the pair of feeding rollers 311b. The pair of feeding rollers 311b feeds out the film 75 to the film holder 331 disposed at the film holding position P11.

The cutter mechanism 312 cuts the film 75 fed out from the pair of feeding rollers 311b to separate the film 75 fed to the film holder 331 from the film roll FR. As shown in FIGS. 12C to 12D, the cutter mechanism 312 includes: a cutter table 312a disposed beneath the film 75; a blade 312b disposed on the cutter table 312a; and a cutter drive part 312c which reciprocates the blade 312b. For example, the cutter drive part 312c, by means of pressure from the pressure source 301, moves the blade 312b in the Y direction so as to cross the film 75 on the cutter table 312a. Thus, the film 75 is cut.

As shown in FIG. 8, the container holding mechanism 320 includes the container holder 321, an opening/closing drive part 322, and the up-down drive part 323. As shown in FIG. 11, the container holder 321 is disposed at the sealing position P3 on the transport path of the transport device 131. That is, when the film holder 331 is disposed at the position P12 above the sealing position P3, the film holder 331 is located at a position immediately above the container holder 321. The container holder 321 holds a specimen container 80 on the specimen rack 85 transported to the sealing position P3, and takes out the specimen container 80 from the specimen rack 85 or returns the specimen container 80 to the specimen rack 85.

The container holder 321 is composed of a pair of hand members 321a for holding a specimen container 80. The pair of hand members 321a are disposed opposing each other so that the specimen container 80 can be placed therebetween. The opening/closing drive part 322 (see FIG. 8) opens and closes the pair of hand members 321a. The opening/closing drive part 322 causes the pair of hand members 321a to hold the specimen container 80 by moving them in a direction in which they approach each other. The opening/closing drive part 322 causes the pair of hand members 321a to release the specimen container 80 by moving them in a direction in which they move away from each other. The up-down drive part 323 is configured to move the pair of hand members 321a in the up-down direction.

The sealing mechanism 330 (see FIG. 8) includes a film holder 331, an opening/closing drive part 332, a rotation drive part 333, and a horizontal drive part 334.

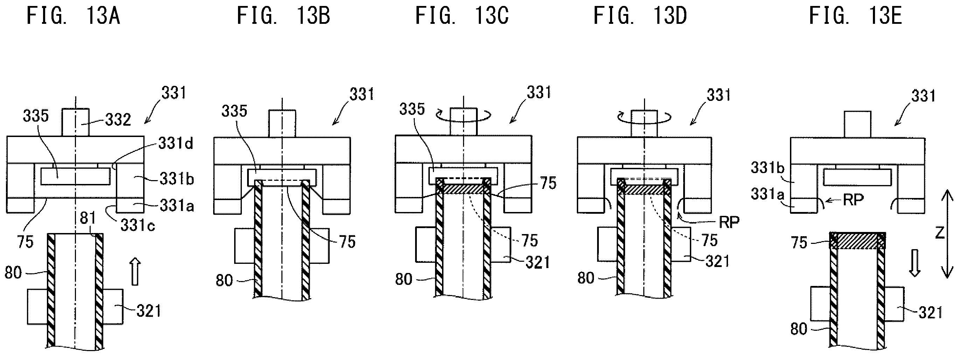

The film holder 331 holds a film piece supplied from the film piece supply mechanism 310. As shown in FIG. 13A, the film holder 331 includes upper and lower members (first member 331a, second member 331b) each having a space in a center portion thereof. The distance between these members is changeable. The distance between the lower first member 331a and the upper second member 331b is increased (see FIG. 12B), and the film 75 is supplied to the space between them. Then, the film 75 is sandwiched, at its periphery, by the first member 331a and the second member 331b by reducing the distance between the first member 331a and the second member 331b (see FIG. 12C), whereby the film 75 is held. The first member 331a has a through-hole 331c in a center portion thereof. The second member 331b has a recess 331d which is opened downward and is communicated with the through-hole 331c of the first member 331a. The through-hole 331c and the recess 331d provide a center space in which an upper end portion of the specimen container 80 is to be inserted.

In the configuration example shown in FIGS. 13A to 13E, the sealing device 300 includes a pressing part 335 which pushes the film 75 covering the opening 81 of the specimen container 80 into the opening 81. Thus, the sealing process can be completed with the film 75 being pushed into the specimen container 80 by the pressing part 335 (see FIG. 13C). Depending on the urine specimen 90, microorganisms in the specimen may generate a gas. Even in such a case, since the internal volume of the specimen container 80 sealed with the film 75 is reduced in advance, the portion of the film 75 pushed into the specimen container 80 is pushed and retreated, thereby preventing an increase in pressure inside the specimen container 80 due to the generated gas. As a result, sealing can be prevented from loosening due to an increase in pressure, and discharge of the gas when the film 75 is punctured can be reduced.

The pressing part 335 is disposed at the inner side of the recess 331d of the second member 331b so as to oppose the held film piece. The pressing part 335 has a shape protruding from the second member 331b toward the first member 331a (protruding downward). The pressing part 335 is formed of an elastic body, and is rotatably mounted to the second member 331b. Examples of the elastic body include rubber, sponge, and elastomer. When the opening 81 of the specimen container 80 is sealed (see FIG. 13C), the pressing part 335 is pressed against the opening edge. The pressing part 335, against which the opening edge is pressed, elastically deforms and partially enters the opening 81 via the film 75. As a result, the portion of the film 75 covering the opening 81 is pushed into the opening 81, adheres to the inner surface of the opening 81, and seals the opening 81.

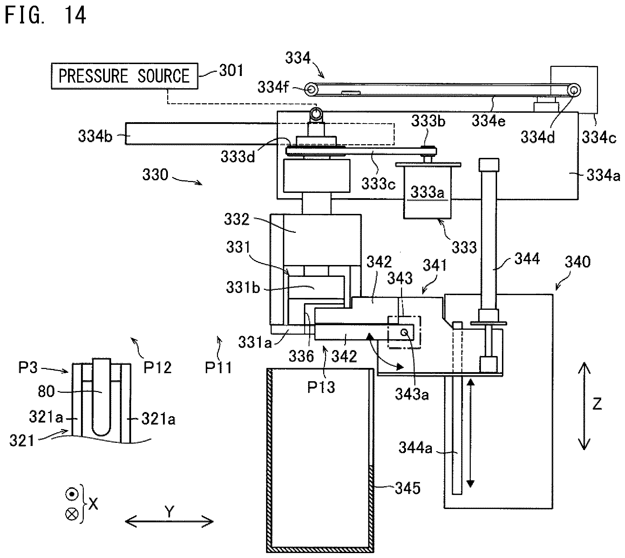

As shown in FIG. 14, the opening/closing drive part 332 of the sealing mechanism 330 (see FIG. 8) opens and closes the film holder 331 by means of pressure from a pressure source 301. The opening/closing drive part 332 causes the first member 331a and the second member 331b to hold the film piece by moving them in a direction in which they approach each other. The opening/closing drive part 332 causes the first member 331a and the second member 331b to release the film piece by moving them in a direction in which they move away from each other. In the configuration example shown in FIG. 14, the opening/closing drive part 332 moves the second member 331b in the up-down direction (Z direction) with respect to the first member 331a.

The rotation drive part 333 causes a motor 333a to rotate a driving pulley 333b attached to an output shaft of the motor 333a, thereby rotating, via a belt 333c, a driven pulley 333d connected to the film holder 331. Thus, the rotation drive part 333 causes the film holder 331 including the first member 331a and the second member 331b to rotate around a center axis of the specimen container 80 (i.e., a vertical axis along the Z direction).

The horizontal drive part 334 has a holding member 334a that holds the film holder 331 so as to be horizontally movable on a rail 334b. The rail 334b extends along the Y direction. The horizontal drive part 334 causes a motor 334c to rotate a driving pulley 334d attached to an output shaft of the motor 334c, thereby rotating a belt 334e between the driving pulley 334d and a driven pulley 334f. The holding member 334a is connected to the belt 334e. Thus, the horizontal drive part 334 horizontally moves the film holder 331 and the rotation drive part 333 held by the holding member 334a to the position P12 above the specimen container 80 held by the container holder 321 (i.e., immediately above the sealing position P3), the film holding position P11, and the film disposal position P13.

In the present embodiment, a portion, of the film 75 held by the film holder 331 (see FIG. 13), which is wound around the specimen container 80 is separated from a residual portion of the film 75. The residual portion of the film 75 is a portion sandwiched by the first member 331a and the second member 331b.

Specifically, the controller (133, 163) (see FIG. 8) controls the rotation drive part 333 so as to operate until the film 75 wound around the specimen container 80 is separated from the portion held by the film holder 331.

Thus, a tensile fracture of the film 75 is caused by rotation, and the film 75 wound around the specimen container 80 can be separated from the film holder 331. As a result, both sealing of the opening 81 with the film 75 and removal of the residual portion of the sealed film 75 can be realized by rotation which is a simple operation. Since the excess film 75 is removed from the specimen container 80, handling of the sealed specimen container 80 is facilitated.

Specifically, firstly, as shown in FIGS. 13A and 13B, the controller 133 (see FIG. 8) controls the up-down drive part 323 so as to drive the container holding mechanism 320 (see FIG. 11) such that an upper end portion of the specimen container 80 passes the through-hole 331c and moves above an upper surface of the first member 331a. Thus, the film 75 is adhered to the opening edge of the specimen container 80 while the film 75 is pressed against the opening edge by its tension. The specimen container 80 is moved upward to a position where the opening edge presses and elastically deforms the pressing part 335.

As shown in FIG. 13C, the controller 133 controls the rotation drive part 333 (see FIG. 8) so as to rotate the film holder 331 such that the film 75 is wound around the specimen container 80 while the film 75 is in contact with the opening edge. Thus, the film 75 covering the opening 81 is wound around the outer peripheral surface of the specimen container 80.

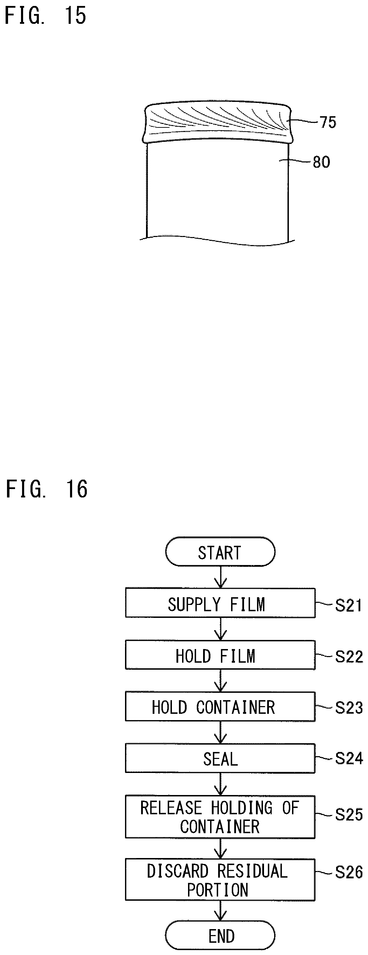

As shown in FIG. 13D, the controller 133 further operates the rotation drive part 333 to rotate the film holder 331. Due to the rotation, the film 75 wound around the specimen container 80 causes a tensile fracture between the outer peripheral surface of the specimen container 80 and the portion of the film 75 being held (the portion sandwiched by the first member 331a and the second member 331b), and is separated from the portion thereof not wound around the specimen container 80. As a result, only the portion, of the film piece held by the film holder 331, which is wound around the specimen container 80 is attached to the specimen container 80 while the residual portion RP remains being held by the film holder 331. FIG. 15 shows a state where the opening 81 is sealed with the film 75 from which the residual portion RP has been separated.

Thereafter, as shown in FIG. 13E, the controller 133 controls the up-down drive part 323 (see FIG. 8) such that the specimen container 80 is moved downward by the container holding mechanism 320 and returned to the specimen rack 85.

As described above, in the configuration of separating the residual portion RP of the film 75 from the specimen container 80, the residual portion RP (see FIG. 13E) is left on the film holder 331. Therefore, the sealing device 300 shown in FIG. 8 includes a film removal part 341 (see FIG. 8) which removes, from the film holder 331, the portion (residual portion RP), of the film 75, which is held by the film holder 331.

Thus, the residual portion RP, of the film 75, which has been separated from the specimen container 80 and held on the film holder 331 side, can be removed by the film removal part 341, whereby the subsequent sealing process can be readily performed.

As shown in FIG. 8, the film removal part 341 is included in the film piece disposal mechanism 340. The film piece disposal mechanism 340 further includes a disposal part 345 in which the residual portion RP is discarded. The film piece disposal mechanism 340 removes the residual portion RP of the film 75 from the film holder 331 located at the film disposal position P13.

With the first member 331a of the film holder 331 being apart from the second member 331b, the film removal part 341 catches a part of the residual portion RP of the film 75 and brings the residual portion RP to the disposal part 345, thereby discarding the residual portion RP.

As shown in FIG. 14, the film removal part 341 includes a pair of sandwiching members 342, a sandwiching drive part 343, and an up-down drive part 344. The pair of sandwiching members 342 are arranged in the up-down direction, and the distance between them is changeable. In FIG. 14, of the pair of sandwiching members 342, an upper member is fixed and a lower member is rotatable around a rotation shaft 343a disposed at an end thereof. The sandwiching drive part 343 is implemented by a motor which rotates the lower member of the pair of sandwiching members 342 around the rotation shaft 343a in the horizontal direction. The sandwiching drive part 343 reduces the distance between the upper member and the lower member such that the residual portion RP of the film 75 is sandwiched by the upper member and the lower member. The sandwiching drive part 343 increases the distance between the upper member and the lower member so as to release the residual portion RP from the pair of sandwiching members 342. The up-down drive part 344 is implemented by an air cylinder, for example. The air cylinder includes a rod, and moves the pair of sandwiching members 342 and the sandwiching drive part 343 along a rail 344a extending in the up-down direction (Z direction) through advancement and retraction of the rod. The disposal part 345 is a disposal box having an open top, and is disposed beneath the pair of sandwiching members 342.

In the first member 331a and the second member 331b of the film holder 331, a cutout 336 is formed such that a part of the residual portion RP of the film 75 is exposed. When the film holder 331 is located at the film disposal position P13 with the distance between the pair of sandwiching members 342 being increased, the residual portion RP of the film 75 exposed from the cutout 336 of the film holder 331 is placed between the pair of sandwiching members 342.

At this time, the controller 133 controls the opening/closing drive part 332 so as to increase the distance between the first member 331a and the second member 331b such that holding by the film holder 331 is released, and controls the sandwiching drive part 343 so as to close the pair of sandwiching members 342 such that the film removal part 341 grasps the residual portion RP. Then, the controller 133 controls the up-down drive part 344 so as to move the pair of sandwiching members 342 downward to the disposal part 345. When the pair of sandwiching members 342 sandwiching the residual portion RP moves downward, the residual portion RP is removed from the film holder 331. Thereafter, the controller 133 controls the sandwiching drive part 343 so as to open the pair of sandwiching members 342 that has entered the disposal part 345. Thus, the residual portion RP having been sandwiched by the pair of sandwiching members 342 is collected into the disposal part 345.

### <Sealing process>

The operation flow of the above sealing process will be described with reference to FIG. 16. The controller 133 performs driving control for each driving source of each mechanism.

In step S21, the controller 133 performs control of causing the film piece supply mechanism 310 to supply the film 75 to the film holder 331. The controller 133 controls the horizontal drive part 334 so as to locate the film holder 331 at the film holding position P11. The controller 133 controls the roller drive part 311c so as to feed the film 75 to a space between the first member 331a and the second member 331b of the film holder 331. The controller 133 controls the cutter drive part 312c so as to cut the fed film 75 and separate the same from the film roll FR.

In step S22, the film 75 is held. The controller 133 controls the opening/closing drive part 322 such that the film 75 supplied from the film piece supply mechanism 310 is sandwiched by the first member 331a and the second member 331b.

In step S23, the specimen container 80 is held. The controller 133 controls the opening/closing drive part 322 such that the pair of hand members 321a holds a specimen container 80 in the specimen rack 85 transported to the sealing position P3. The controller 133 controls the horizontal drive part 334 so as to locate the film holder 331 to the position P12 above the sealing position P3. The controller 133 controls the up-down drive part 323 such that the pair of hand members 321a holding the specimen container 80 is moved upward and placed in a space (corresponding to the through-hole 331c and the recess 331d) in the film holder 331. Thus, the opening edge at the upper end of the specimen container 80 is adhered to the film 75, and the film 75 is pushed into the opening 81 by the pressing part 335 (see FIG. 13B).

In step S24, the opening 81 is sealed. The controller 133 controls the rotation drive part 333 so as to rotate the film holder 331 such that the film 75 held by the film holder 331 is wound around the outer peripheral surface of the specimen container 80. The controller 133 operates the rotation drive part 333 until the film 75 wound around the specimen container 80 is separated from the film 75 held by the film holder 331. Thus, the specimen container 80 is sealed with the film 75, and the film 75 wound around the specimen container 80 is separated from the residual portion RP of the film 75 held by the film holder 331 (see FIG. 13D).

In step S25, holding of the specimen container 80 is released. The controller 133 controls the up-down drive part 323 so as to move the pair of hand members 321a holding the specimen container 80 downward, and return the sealed specimen container 80 to the specimen rack 85. The controller 133 controls the opening/closing drive part 322 so as to increase the distance between the pair of hand members 321a, and release holding of the specimen container 80 by the pair of hand members 321a. After the holding is released, the transport controller 132 controls the transport device 131 so as to transport the specimen rack 85 such that a specimen container 80 to be sealed next is located at the sealing position P3.

In step S26, the residual portion RP is discarded. The controller 133 controls the horizontal drive part 334 such that the film holder 331 holding the residual portion RP of the film 75 is located at the film disposal position P13. The controller 133 controls the opening/closing drive part 332 so as to increase the distance between the first member 331a and the second member 331b, and controls the sandwiching drive part 343 so as to close the pair of sandwiching members 342. Thus, the residual portion RP of the film 75 is sandwiched by the pair of sandwiching members 342 of the film removal part 341. The controller 133 controls the up-down drive part 344 so as to move the pair of sandwiching members 342 downward to the disposal part 345. Thereafter, the controller 133 controls the sandwiching drive part 343 so as to open the pair of sandwiching members 342 which has entered the disposal part 345. Thus, the residual portion RP of the film 75 having been sandwiched by the pair of sandwiching members 342 is collected into the disposal part 345.

### (Transport control for specimen rack)

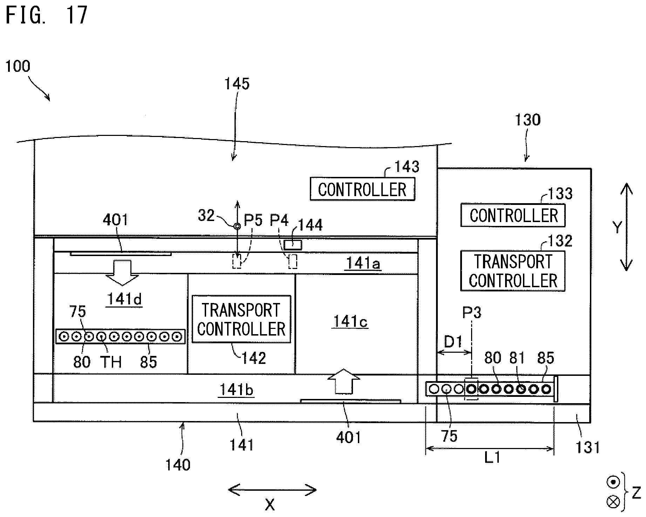

When all the specimen containers 80 to be sealed have been subjected to the sealing process, the specimen rack 85 is transported from the sealing unit 130 to the specimen processing unit 140 (see FIG. 1). In the example shown in FIG. 17, the sealing unit 130 is configured such that a distance D1 from an outlet of the sealing unit 130 to the sealing position P3 is shorter than a length L1 of the specimen rack 85. In this case, the distance D1 being reduced enables reduction in the width of the sealing unit 130 in the transport direction (X direction) of the specimen rack 85.

For example, in FIG. 17, if the fourth specimen container 80, from the head of the specimen rack 85 capable of holding ten specimen containers 80, is located at the sealing position P3, the head of the specimen rack 85 enters the transport device 141 of the specimen processing unit 140.

The transport device 141 of the specimen processing unit 140, by using a movable member 401, moves the received specimen rack 85 from the second transport path 141b having an inlet to the loading/storage part 141c, and further moves the specimen rack 85 from the loading/storage part 141c to the first transport path 141a. Since aspiration of the urine specimen 90 from each specimen container 80 (see FIGS. 7A to 7C) is performed at the aspiration position P5 above the first transport path 141a, one or more specimen racks 85 wait in the loading/storage part 141c when specimen processing is continuously performed.

Therefore, in the sealing process of the sealing unit 130, if the specimen rack 85 enters the transport device 141 of the specimen processing unit 140, the transport device 141 cannot transport the specimen rack 85 from the second transport path 141b to the loading/storage part 141c by using the movable member 401.

In the present embodiment, the transport controller 132 of the sealing unit 130 is configured to perform control of transporting the specimen rack 85 such that the K-th and subsequent specimen holding positions from the head of the specimen rack 85 are transported to the sealing position P3, on the condition that the transport device 141 can receive the specimen rack 85. The "K-th" indicates a specimen holding position that causes the head of the specimen rack 85 to enter the inlet of the transport device 141 if the K-th specimen holding position is located at the sealing position P3. The transport controller 132 is configured to perform control of suspending transport of the K-th and subsequent specimen holding positions from the head of the specimen rack 85 to the sealing position P3 if the transport device 141 cannot receive the specimen rack 85. FIG. 17 shows a case where K=4, for example.

The state where the transport device 141 can receive the specimen rack 85 is the state where no specimen rack 85 is placed in the loading/storage part 141c. The state where the transport device 141 cannot receive the specimen rack 85 is the state where a specimen rack 85 is placed in the loading/storage part 141c. The transport controller 132 acquires the transport state of the specimen rack 85 in the transport device 141 from the transport controller 142 of the specimen processing unit 140. The transport controller 132 inhibits transport of the K-th and subsequent specimen holding positions to the sealing position P3 when a specimen rack 85 is placed in the loading/storage part 141c of the transport device 141, and permits transport of the K-th and subsequent specimen holding positions to the sealing position P3 when no specimen rack 85 is placed in the loading/storage part 141c.

### (Specimen processing device)

Next, the specimen processing devices included in the specimen processing unit 140 and the specimen processing unit 150 shown in FIG. 2 will be described.

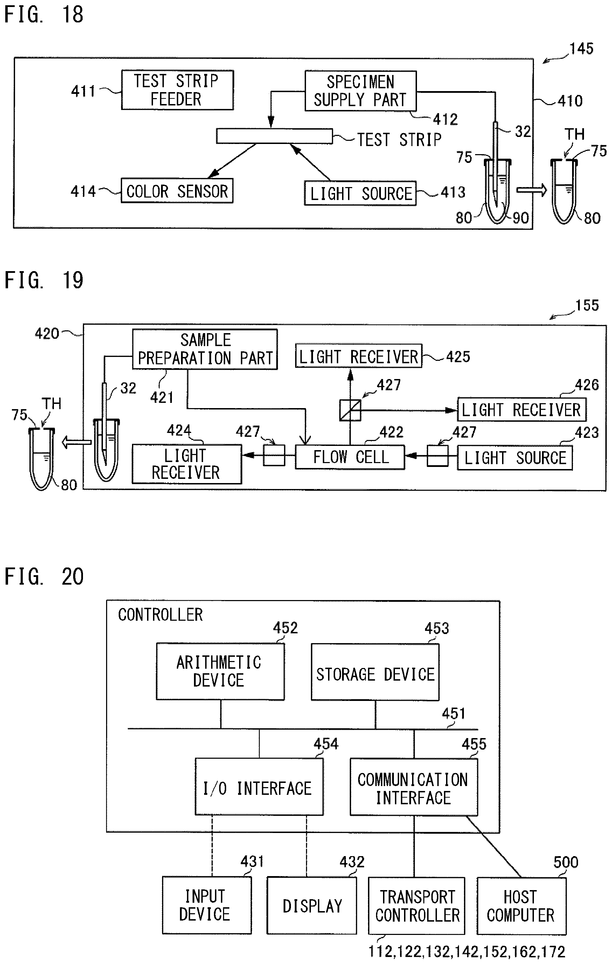

The specimen processing device 145 of the specimen processing unit 140 performs measurement related to a urine qualitative test. The urine qualitative test is a test for measuring chemical components in urine related to a laboratory test. As shown in FIG. 18, the specimen processing device 145 includes a measurement part 410 which performs measurement of a urine specimen by detecting a color of a test strip on which the urine specimen is applied.

The measurement part 410 applies the urine specimen to the test strip, and measures color reactions on the test strip, thereby measuring test item components contained in the urine specimen. The measurement part 410 feeds out a test strip from a test strip feeder 411 in which test strips are stored, to a predetermined test position inside the measurement part 410. A specimen supply part 412 supplies a urine specimen 90 aspirated from a specimen container 80 by the aspiration tube 32, to the test strip. The measurement part 410 irradiates the test strip with measurement light emitted from a light source 413, and receives the measurement light by a color sensor 414 to measure color reactions on the test strip. Examples of measurement items include glucose, protein, bilirubin, and ph (hydrogen ion exponent).

The controller 143 of the specimen processing unit 140 controls the specimen processing device 145 such that, when the aspiration tube 32 aspirates the urine specimen 90, agitation by aspiration is performed in the specimen container 80 by using the aspiration tube 32 which has penetrated through the film 75. The agitation by aspiration is a process of agitating the urine specimen 90 through aspiration of the urine specimen 90 from the specimen container 80 and discharge of the aspirated urine specimen 90 into the specimen container 80. The agitation by aspiration can reduce leakage of the urine specimen 90 from the specimen container 80 during agitation, as compared to, for example, agitation by inversion in which the urine specimen 90 is agitated by inverting the specimen container 80. Therefore, the unpleasant feeling of the user due to odor of the urine specimen 90 can be effectively reduced.

The specimen processing device 155 of the specimen processing unit 150 performs measurement related to a urinary sediment test. The urinary sediment test is a test for measuring solid components (sediments) in urine, and classifying and counting the solid components. As shown in FIG. 19, the specimen processing device 155 includes a measurement part 420 which measures solid components in a urine specimen by a flow cytometry method.

The measurement part 420 includes a flow cytometer. In the measurement part 420, a sample preparation part 421 prepares a measurement sample by using a reagent such as a staining liquid, from the urine specimen 90 aspirated from the specimen container 80 by the aspiration tube 32. The controller 153 of the specimen processing unit 150 controls the specimen processing device 155 such that, when the urine specimen 90 is aspirated, agitation by aspiration is performed in the specimen container 80 by the aspiration tube 32 which has penetrated through the film 75. Then, the sample preparation part 421 supplies the measurement sample to a flow cell 422. The light source 423 irradiates the flow cell 422 with measurement light. A light receiver 424, a light receiver 425, and a light receiver 426 respectively measure forward scattered light, side scattered light, and fluorescence, of the measurement light, generated from solid components in the measurement sample that flows in the flow cell 422. An optical system 427 including a lens, a spectrometer, etc., is disposed between the flow cell 422 and each of the light source 423, the light receiver 424, the light receiver 425, and the light receiver 426. The controller 153 counts and classifies the solid components by analyzing obtained light reception signals. Examples of the solid components include red blood cells, white blood cells, epithelial cells, casts, and bacteria contained in the urine specimen 90.

### (Controller)

FIG. 20 is a block diagram showing a configuration example of a computer usable as a controller (controllers 113 to 163). The computer includes an arithmetic device 452, a storage device 453, an I/O interface 454, and a communication interface 455 which are mutually connected via a bus 451. The arithmetic device 452 is a processor. The storage device 453 includes a semiconductor storage element and/or a hard disk drive. The storage device 453 stores therein various kinds of programs for causing the computer to operate as the controller. The arithmetic device 452 performs commands included in the programs stored in the storage device 453 to cause the computer to function as the controller. The I/O interface 454 is an interface for connecting the computer with external equipment. The communication interface 455 is an interface through which the controller communicates with external devices including the host computer 500.

The controller 143 of the specimen processing unit 140 and the controller 153 of the specimen processing unit 150 are configured to analyze data obtained from the corresponding specimen processing devices 145 and 155, respectively. An input device 431 through which the user inputs various kinds of information to the computer and a display 432 on which the computer displays various kinds of information can be connected to the I/O interface 454 of each of the controller 143 and the controller 153. The input device 431 and the display 432 may be built in the computer, or may be (externally) connected to the computer. For example, the input device 431 may be a keyboard, a mouse, a touch sensor, or the like. The display 432 is a display device such as a liquid crystal monitor. The display 432 may be a touch panel display integrated with a touch sensor as the input device 431.

### (Flow of specimen processing method)

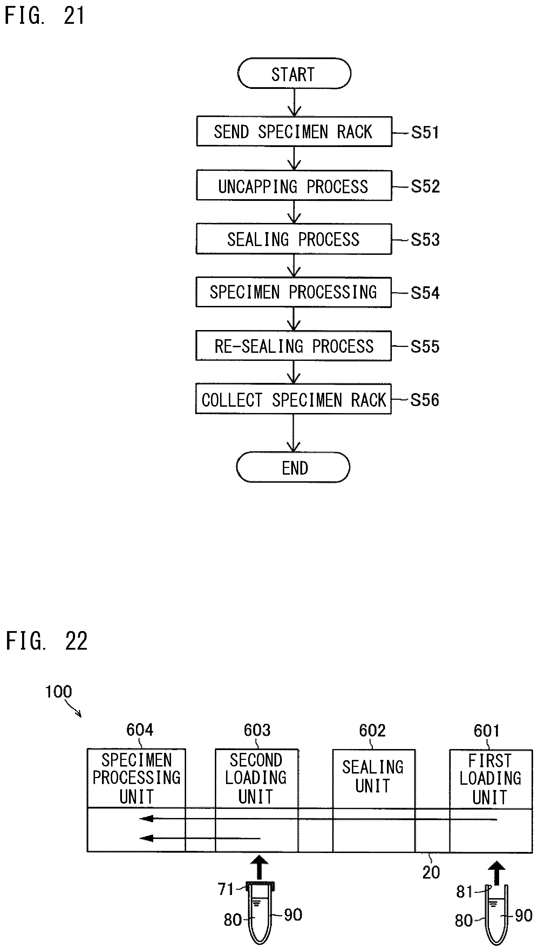

With reference to FIG. 21, a flow of a specimen processing method using the specimen processing system 100 will be described. As for the configuration of the specimen processing system 100, FIG. 1 and FIG. 2 are referred to.

Firstly, a specimen rack 85 in which specimen containers 80 to be processed are set is placed in the loading unit 110 by the user. When the user has performed an input operation on a start button, specimen processing is started.

In step S51, a transport step for the specimen rack 85 is performed. The controller 113 of the loading unit 110 (see FIG. 1) controls the information reader 114 so as to read identification information attached to each specimen container 80. Moreover, the transport controller 112 controls the transport device 111 so as to sequentially transport a plurality of specimen containers 80 in the specimen rack 85 to the reading position P1, and thereafter transport the specimen rack 85 to the uncapping unit 120.

In step S52, an uncapping step is performed by the uncapping unit 120. The transport controller 122 controls the transport device 121 so as to receive the specimen rack 85 from the loading unit 110 and sequentially transport the plurality specimen containers 80 in the specimen rack 85 to the uncapping position P2. During the transport of the specimen containers 80 to the uncapping position P2, the controller 123 controls the container identification part 201 (see FIG. 3) so as to identify a cap 84 of each specimen container 80, and determines whether or not the specimen container 80 is a specimen container to be uncapped, on the basis of an identification result of the container identification part 201. The controller 123 controls the uncapping device 124 so as to selectively perform an uncapping process at the uncapping position P2 on the specimen container 80 to be uncapped. The transport controller 122 controls the transport device 121 so as to transport the specimen rack 85 to the sealing unit 130 after all the specimen containers 80 set in the specimen rack 85 have been subjected to the uncapping process.

In step S53, a sealing step is performed by the sealing unit 130 (see FIG. 1). The transport controller 132 controls the transport device 131 so as to receive the specimen rack 85 from the uncapping unit 120 and sequentially transport the plurality of specimen containers 80 in the specimen rack 85 to the sealing position P3. The control for transporting the specimen containers 80 to the sealing position P3 is performed on the basis of a distance of each specimen container 80 to the sealing position P3 and a feeding amount per pulse of a motor, when reception of the specimen rack 85 from the uncapping unit 120 to the sealing unit 130 has been detected. The controller 133 controls the sealing device 300 so as to perform the sealing process at the sealing position P3. The transport controller 132 controls the transport device 131 so as to transport the specimen rack 85 to the specimen processing device 145 when all the specimen containers 80 set in the specimen rack 85 have been subjected to the sealing process.

In step S54, a specimen processing step is performed by the specimen processing unit 140 and the specimen processing unit 150 (see FIG. 1). Firstly, in the specimen processing unit 140, the transport controller 142 controls the transport device 141 so as to receive the specimen rack 85 from the sealing unit 130 and transport the plurality of specimen containers 80 in the specimen rack 85 from the second transport path 141b to the first transport path 141a. The transport device 141 sequentially transports the plurality of specimen containers 80 in the specimen rack 85 to the reading position P4, and thereafter sequentially transports the specimen containers 80 to the aspiration position P5. The controller 143 controls the information reader 144 so as to read identification information attached to each specimen container 80 at the reading position P4. On the basis of the read identification information, the controller 143 acquires, from the host computer 500, a measurement order for the urine specimen 90 contained in each specimen container 80. As for a specimen container 80 that is set as a test target according to the measurement order, the controller 143 causes the aspiration tube 32 to aspirate the urine specimen 90 from the specimen container 80 at the aspiration position P5. The controller 143 controls the specimen processing device 145 so as to perform a specimen measurement operation, and analyzes measurement data obtained from the specimen processing device 145. The transport controller 142 controls the transport device 141 so as to transport the specimen rack 85 for which aspiration has been completed, from the first transport path 141a to the second transport path 141b, and transport the specimen rack 85 from the second transport path 141b to the specimen processing unit 150.

In the specimen processing unit 150, the transport controller 152 controls the transport device 151 so as to receive the specimen rack 85 from the specimen processing unit 140 and transport the plurality of specimen containers 80 in the specimen rack 85 from the second transport path 151b to the first transport path 151a. The transport device 151 sequentially transports the plurality of specimen containers 80 in the specimen rack 85 to the reading position P6, and thereafter sequentially transports the specimen containers 80 to the aspiration position P7. The controller 153 controls the information reader 154 so as to read the identification information attached to each specimen container 80 at the reading position P6. On the basis of the read identification information, the controller 153 acquires, from the host computer 500, a measurement order for the urine specimen 90 contained in each specimen container 80. As for a specimen container 80 that is set as a test target according to the measurement order, the controller 153 causes the aspiration tube 32 to sequentially aspirate the urine specimen 90 from the specimen container 80 at the aspiration position P7. The controller 153 controls the specimen processing device 155 so as to perform a specimen measurement operation, and analyzes measurement data obtained from the specimen processing device 155. The transport controller 152 controls the transport device 151 so as to transport the specimen rack 85 for which aspiration has been completed, from the first transport path 151a to the second transport path 151b, and transport the specimen rack 85 from the second transport path 151b to the second sealing unit 160.

The specimen processing unit 150 performs specimen measurement not only when an order of a urinary sediment test has been registered in advance but also when an order of a urinary sediment test is registered on the basis of an analysis result of a urine qualitative test performed by the upstream-side specimen processing unit 140. The controller 153 controls the operation of the specimen processing device 155 such that specimen aspiration is not performed on a specimen container 80 for which specimen processing (urinary sediment test) by the specimen processing unit 150 is not required according to measurement order information.

In step S55, a re-sealing step is performed by the second sealing unit 160. The transport controller 162 controls the transport device 161 so as to receive the specimen rack 85 from the specimen processing unit 150, and sequentially transport the plurality of specimen containers 80 in the specimen rack 85 to the sealing position P8.

In the present embodiment, the step of re-sealing a specimen container 80 is selectively performed on the basis of identification information. That is, the controller 163 acquires identification information having been read by at least one of the information reader 114, the information reader 144, and the information reader 154 through inter-device communication, and individually identifies the specimen containers 80 in the specimen rack 85. The controller 163 is configured to control the second sealing unit 160 so as to selectively perform re-sealing on the specimen containers 80 on the basis of the read identification information.

Thus, sealing of the specimen containers 80 can be selectively performed in accordance with difference in processes to be performed on the urine specimens 90 having been subjected to specimen processing. For example, re-sealing is performed on a specimen container 80 whose urine specimen 90 is planned to be preserved or transferred to another department, while re-sealing is not performed on a specimen container 80 whose urine specimen 90 is to be discarded.

The controller 163 acquires, from the host computer 500, information related to a process for a urine specimen 90 on the basis of identification information of the urine specimen 90. For example, the controller 163 selects to perform re-sealing on a specimen container 80 that contains a urine specimen 90 to be preserved or transferred to another test department. For example, the controller 163 selects not to perform re-sealing on a specimen container 80 that contains a urine specimen 90 to be discarded.

The controller 163 controls the sealing mechanism (sealing device 300) so as to perform the sealing process at the sealing position P8. The transport controller 162 controls the transport device 161 so as to transport the specimen rack 85 to the collection unit 170 when the specimen containers 80 to be re-sealed, set in the specimen rack 85, have been subjected to the sealing process.

In step S56, a collection step for the specimen rack 85 is performed. The transport controller 172 of the collection unit 170 controls the transport device 171 so as to receive the specimen rack 85 from the second sealing unit 160, and transport the received specimen rack 85 to the storage position.

Thus, execution of the specimen processing method by the specimen processing system 100 is completed for one specimen rack 85. When the user sets a plurality of specimen racks 85 in the loading unit 110, the aforementioned steps are sequentially performed for each of the specimen racks 85.

The specimen rack 85 transported to the storage position is collected by the user. The urine specimens 90 having been analyzed and collected are subjected to post-processing such as disposal or preservation, according to the test results or circumstances. For example, the user judges whether or not a specimen container 80 having been subjected to specimen analysis needs to be preserved for a retest or an additional test. A specimen container 80 whose urine specimen need not be preserved is transported to a disposal place and discarded by a predetermined method. A specimen container 80 whose urine specimen needs to be preserved for a predetermined period or transported to another department is preserved for the predetermined period at a predetermined place under a predetermined environment (refrigerating storage, or room-temperature storage in some cases). The user re-loads the preserved specimen container 80 to the specimen processing system 100 at a desired timing to perform a retest. Alternatively, the preserved specimen container 80 is subjected to another test in another test system. If abnormality is detected in a urine specimen 90 as a result of the analysis, this urine specimen 90 may be subjected to a bacteriological test for diagnosis of urinary tract infection.

### (Modifications)

It should be noted that the embodiment disclosed herein is merely illustrative in all aspects and should not be considered as being restrictive. The scope of the present disclosure is not defined by the description of the above embodiment but by the scope of the claims, and further includes meaning equivalent to the scope of the claims and all changes (modifications) within the scope of the claims.

For example, in the embodiment described above, one loading unit 110 is disposed. However, a plurality of loading units 110 may be disposed. In the loading unit 110, all the specimen containers 80 have the caps 84 attached thereto. However, specimen containers 80 having caps 84 and specimen containers 80 having no caps 84 may coexist.

For example, in an example shown in FIG. 22, a specimen processing system 100 includes a first loading unit 601 and a second loading unit 603 each for receiving a specimen container 80. A transport device 20 transports the specimen container 80 in the first loading unit 601 toward a specimen processing unit 604 through a sealing unit 602, and transports the specimen container 80 in the second loading unit 603 to the specimen processing unit 604 not through the sealing unit 602. In this example, the first loading unit 601, the sealing unit 602, the second loading unit 603, and the specimen processing unit 604 are arranged in this order along the transport path of the transport device 20. The sealing unit 602 seals the opening 81 of the specimen container 80 received from the first loading unit 601 with a seal 71.

In the above configuration, when a specimen container 80 to be sealed and a specimen container 80 not to be sealed are present, the specimen container 80 to be sealed can be set in the first loading unit 601 while the specimen container 80 not to be sealed can be set in the second loading unit 603. The specimen container 80 set in the first loading unit 601 can be sealed by the sealing unit 602 while the specimen container 80 set in the second loading unit 603 can be transported not through the sealing unit 602, whereby specimen processing can be efficiently performed.

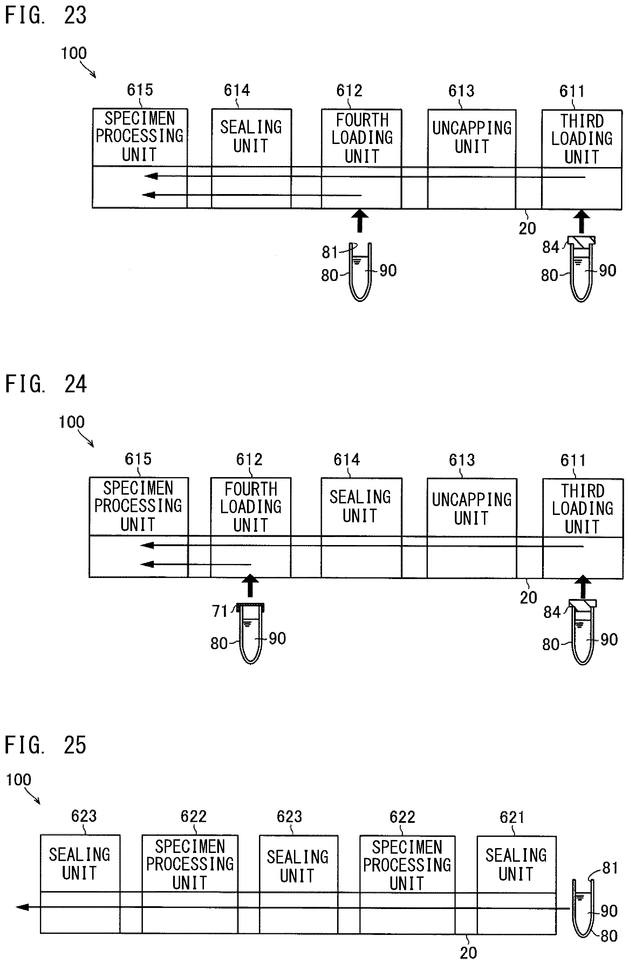

In examples shown in FIG. 23 and FIG. 24, a specimen processing system 100 includes a third loading unit 611 and a fourth loading unit 612 each for receiving a specimen container 80. A transport device 20 transports the specimen container 80 in the third loading unit 611 toward a specimen processing unit 615 through an uncapping unit 613, and transports the specimen container 80 in the fourth loading unit 612 toward the sealing unit 614 or the specimen processing unit 615 not through the uncapping unit 613.

In the configuration shown in FIG. 23, the specimen container 80, in the third loading unit 611, having the cap 84 attached thereto is transported to the uncapping unit 613, the sealing unit 614, and the specimen processing unit 615 in this order. The specimen container 80, in the fourth loading unit 612, whose opening 81 is opened is transported to the sealing unit 614 and the specimen processing unit 615 not through the uncapping unit 613. Meanwhile, in the configuration shown in FIG. 24, a specimen container 80 sealed with a seal 71 in advance is placed in the fourth loading unit 612. The specimen container 80 in the fourth loading unit 612 is transported to the specimen processing unit 615 not through the uncapping unit 613 and the sealing unit 614. The specimen container 80, in the third loading unit 611, having the cap 84 attached thereto is transported to the uncapping unit 613, the sealing unit 614, and the specimen processing unit 615 in this order.

In the configuration examples shown in FIG. 23 and FIG. 24, the specimen container 80 having the cap 84 attached thereto in advance can be set in the third loading unit 611 while the specimen container 80 that does not need removal of a cap 84 can be set in the fourth loading unit 612. The cap 84 can be removed from the specimen container 80 set in the third loading unit 611 by the uncapping unit 613, and the specimen container 80 set in the fourth loading unit 612 can be transported not through the uncapping unit 613, whereby specimen processing can be efficiently performed. A fifth loading unit may be disposed between the uncapping unit 613 and the sealing unit 614, in addition to the third loading unit 611 and the fourth loading unit 612.

In an example shown in FIG. 25, a specimen processing system 100 includes a plurality of specimen processing units 622 and a plurality of second sealing units 623. The plurality of second sealing units 623 perform re-sealing on specimen containers 80 having been subjected to aspiration in different specimen processing units 622. In the example of FIG. 25, after a sealing unit 621, one second sealing unit 623 is disposed immediately after each of two specimen processing units 622.

Thus, specimen processing using one or a plurality of specimen processing units 622 can be performed for one specimen container 80. Since the plurality of second sealing units 623 are disposed, a specimen container 80 having been subjected to specimen processing in any of the specimen processing units 622 can be re-sealed by any of the second sealing units 623, whereby odor of the urine specimen 90 released from the specimen container 80 can be reduced.

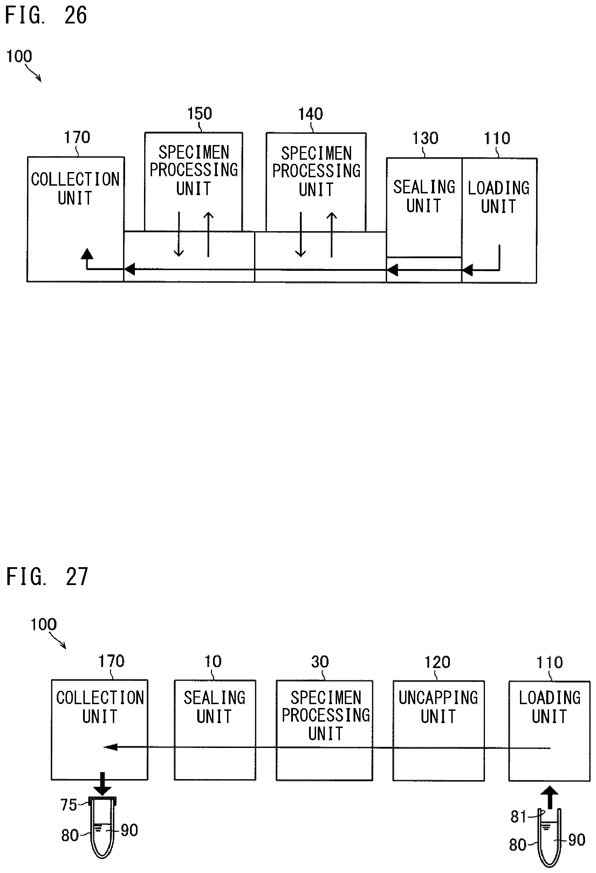

Although the configuration example of the above embodiment includes the uncapping unit 120, the uncapping unit 120 may be dispensed with as shown in FIG. 26. In this case, as for a specimen container 80 having a cap 84 attached thereto, the user may remove the cap 84 before loading the specimen container 80 into the specimen processing system 100. In FIG. 26, the specimen processing system 100 is not provided with an uncapping unit 120 and a second sealing unit 160, and a loading unit 110, a sealing unit 130, a specimen processing unit 140, a specimen processing unit 150, and a collection unit 170 are arranged in this order. In this case, after specimen processing performed by the specimen processing units 140 and 150, the specimen rack 85 may be again transported to the sealing unit 130, and an opening 81 of a specimen container 80 may be re-sealed with a film 75 through which the aspiration tube 32 can penetrate. The specimen processing system 100 may further include a film removal unit for removing the film 75 with which the opening 81 of the specimen container 80 is sealed by the sealing unit 130. A second sealing unit 160 may be disposed between the specimen processing unit 150 and the collection unit 170.

In the above embodiment, an opening 81 of a specimen container 80 is sealed with a film 75 through which the aspiration tube 32 can penetrate, before the specimen is aspirated by the aspiration tube 32 in the specimen processing device 145. However, the present disclosure is not limited thereto. In FIG. 27, no sealing unit 130 is disposed on the upstream side of a specimen processing unit 30, and a loading unit 110, an uncapping unit 120, the specimen processing unit 30, a sealing unit 10, and a collection unit 170 are arranged in this order. Thus, the opening 81 of the specimen container 80 may be sealed, after specimen aspiration, with the seal 71 through which the aspiration tube 32 can penetrate, instead of sealing the opening 81 of the specimen container 80, before specimen aspiration, with the film 75 through which the aspiration tube 32 can penetrate.

### (Third specimen processing method)

A third specimen processing method of the present embodiment is a method of processing a urine specimen 90 contained in a specimen container 80 having an opening 81 by using a specimen processing device 31 as shown in FIG. 28. The method includes (1) a step of aspirating the urine specimen 90 from the specimen container 80 containing the specimen by using an aspiration tube 32, (2) a step of performing specimen processing on the aspirated urine specimen 90, and (3) a step of sealing the opening 81 of the specimen container 80 from which the urine specimen 90 has been aspirated, with a seal 71 through which the aspiration tube 32 can penetrate.

In FIG. 28, in the step (1) of aspirating the urine specimen 90 from the specimen container 80 containing the specimen by using the aspiration tube 32, the opening 81 of the specimen container 80 is not sealed. The urine specimen 90 is aspirated from the specimen container 80, whose opening 81 is not covered but is opened, by using the aspiration tube 32 of the specimen processing device 31.

The step (2) of performing the specimen processing on the aspirated urine specimen 90 is performed by the specimen processing device 31. The processing performed by the specimen processing device 31 is not particularly limited but can be specimen measurement similar to that of the above embodiment.

In the step (3) of sealing the opening 81 of the specimen container 80, from which the urine specimen 90 has been aspirated, with the seal 71 through which the aspiration tube 32 can penetrate, the opening 81 of the specimen container 80 is sealed with the seal 71 for the first time. The sealing process can be performed by the sealing unit 10. The specimen container 80 having been subjected to specimen processing and sealed is subjected to post-processing such as disposal or preservation according to the test results, circumstances, or the like as described above.

According to the third specimen processing method, since the opening 81 is sealed with the seal 71 after aspiration of the urine specimen 90, release of odor from the specimen container 80 can be reduced while the urine specimen 90 is preserved for a retest or the like. When the specimen container 80 sealed with the seal 71 is again subjected to specimen processing for a retest or the like, the aspiration tube 32 can penetrate through the seal 71 and aspirate the urine specimen 90, which makes an uncapping operation unnecessary. Therefore, complication of uncapping and capping operations can be reduced. Moreover, even when an uncapping operation is performed on a specimen container 80 sealed with a cap, the removed cap need not be stored for recapping, thereby reducing complication of uncapping and capping operations.

### (Selection of sealing method)

In the above embodiment, only one example has been described for the sealing method by the sealing device 300. However, the specimen processing method of the present disclosure is not limited thereto, and a sealing method to be performed may be selected from among a plurality of sealing methods in the step of sealing a specimen container with a seal.

Thus, sealing of each specimen container 80 can be performed by a more appropriate sealing method. For example, different sealing methods can be selected according to the specimen type, whether it is a first test or a retest, the content of specimen processing, whether or not preservation is required, the transport destinations, or the like. For example, as for a specimen container 80 to be subjected to agitation by inversion, a sealing method capable of more reliable sealing can be selected. Moreover, different sealing patterns make it possible to discriminate specimen containers 80 by the appearances of the seals 71.

In the above embodiment, the sealing method shown in FIGS. 13A to 13E is regarded as a first sealing method. A film 75 attached to a specimen container 80 by the first sealing method is obtained by winding the film 75 around a portion, of the specimen container 80, from an opening edge to an upper part of an outer peripheral surface as shown in FIG. 15, and removing a residual portion RP of the film 75.

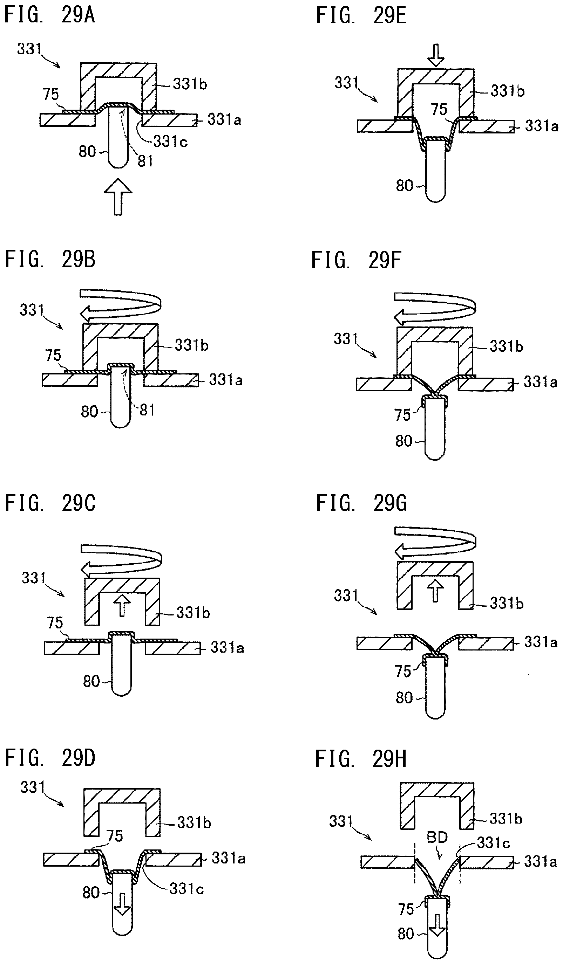

With reference to FIGS. 29A to 29H, a flow of a second sealing method different from the first sealing method will be described.
(A) With a film 75 being held by the film holder 331, an upper end portion of a specimen container 80 is pressed against the film 75 from below via the through-hole 331c of the first member 331a. The film 75 is adhered to the opening edge and the opening 81 is covered with the film 75.
(B) The film holder 331 is rotated at a rotation angle of about 240°. The film 75 is wound around the outer peripheral surface of the specimen container 80.
(C) With the distance between the first member 331a and the second member 331b of the film holder 331 being increased, the film holder 331 is rotated at a rotation angle of about 120°. The film 75, which has been sandwiched and adhered to the lower first member 331a, is removed from the first member 331a.
(D) The specimen container 80 held by the container holder 321 is moved downward. The specimen container 80 is moved downward until an outer peripheral portion of the film 75 is left on the first member 331a and a portion, of the film 75, inside the outer peripheral portion is placed in the through-hole 331c of the first member 331a.
(E) The distance between the first member 331a and the second member 331b is reduced, and the outer peripheral portion of the film 75 present on the first member 331a is sandwiched by the first member 331a and the second member 331b. Thus, in the second sealing method, the portion, of the film 75, held by the film holder 331 is changed during sealing.
(F) The film holder 331 is rotated at a rotation angle of about 240°. The film 75 is wound upward such that it is folded back upward from a lower end of the portion already wound around the specimen container 80 through the first rotation, and is superposed on the already wound portion. On the upper side of the upper end portion of the specimen container 80, a residual portion, of the film 75, which is not wound around the specimen container 80 is twisted and bundled immediately above the opening 81.
(G) With the distance between the first member 331a and the second member 331b of the film holder 331 being increased, the film holder 331 is rotated at a rotation angle of about 120°. The outer peripheral portion, of the film 75, which has been adhered to the lower first member 331a is removed from the first member 331a.
(H) The specimen container 80 held by the container holder 321 is moved downward, and the specimen container 80 is returned to the specimen rack 85. At this time, a bundle BD, of the residual portion of the film 75, which is formed by the rotation passes through the through-hole 331c of the first member 331a, whereby the bundle BD of the residual portion is shaped such that it does not spread in the horizontal direction but rises upward from the specimen container 80. Thereafter, holding of the specimen container 80 by the container holder 321 is released.

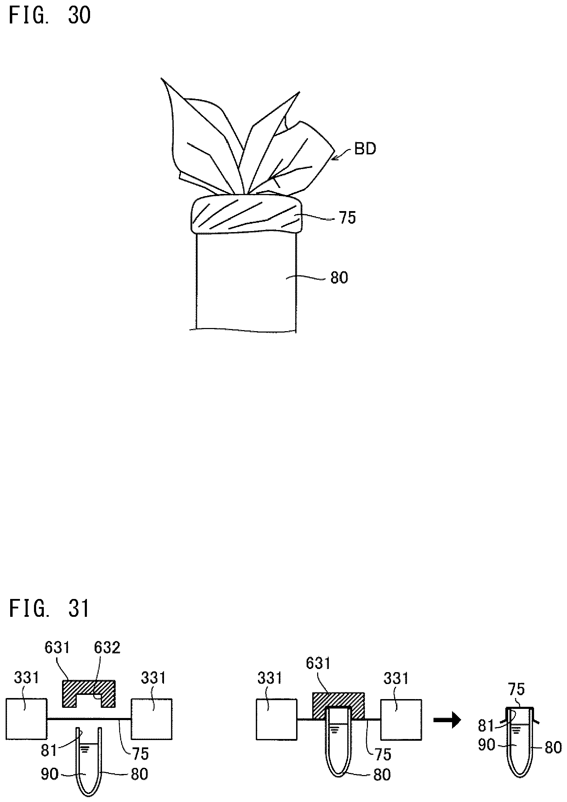

FIG. 30 is a schematic diagram showing the film 75 attached to the specimen container 80 by the second sealing method. The film 75 is folded back and doubly closes the opening 81, and the bundle BD of the residual portion of the film 75 is formed above the specimen container 80. According to the second sealing method, since the residual portion of the film 75 is not separated, the film removal part 341 described in the above embodiment is not required. Since the bundle BD of the residual portion is compactly formed above the specimen container 80, the residual portion of the film 75 does not hinder handling of the specimen container 80. Since the opening 81 of the specimen container 80 is doubly covered with the film 75, sealing performance is enhanced and suitable for preservation. Even when the specimen container 80 is subjected to agitation by inversion, leakage of the urine specimen 90 can be effectively reduced.

For example, in the re-sealing process shown in step S55 in FIG. 21, the controller 163 of the second sealing unit 160 selects a sealing method for a specimen container 80 on the basis of identification information having been read. On the basis of the identification information, the controller 163 controls the sealing device 300 so as to acquire, from the host computer 500, information related to processing for a urine specimen 90 in the specimen container 80, and perform re-sealing according to the first sealing method if the specimen container 80 needs to be subjected to a retest. The controller 163 controls the sealing device 300 so as to perform re-sealing on a specimen container 80 to be preserved in a predetermined place for a predetermined period, and a specimen container 80 to be transported to another test department. The controller 163 may be configured not to perform re-sealing on a specimen container 80 containing a urine specimen 90 to be discarded.

In the above embodiment, in the sealing device 300, the rotation drive part 333 rotates the film holder 331. However, the rotation drive part 333 may rotate the container holder 321, or the rotation drive part 333 may rotate both the film holder 331 and the container holder 321.

In the above embodiment, in the sealing device 300, the rotation drive part 333 is operated until the film 75 wound around the specimen container 80 is separated from the portion of the film 75 held by the film holder 331, whereby the portion of the film 75 wound around the specimen container 80 is separated from the residual portion RP of the film 75. However, the present disclosure is not limited thereto. The film 75 wound around the specimen container 80 may be left as it is. That is, after the film 75 has been wound around the specimen container 80 as shown in FIG. 13C, holding of the film 75 may be released without performing the operations in FIG. 13D and FIG. 13E. In this case, the film 75 covering the opening 81 of the specimen container 80 has a peripheral edge portion (corresponding to the residual portion RP). In this case, since the residual portion RP is not left on the film holder 331, the film removal part 341 can be dispensed with.

In the above embodiment, the up-down drive part 323 drives the container holder 321 in the up-down direction. However, the up-down drive part 323 may drive the film holder 331 in the up-down direction, or may drive both the container holder 321 and the film holder 331 in the up-down direction.

In the above embodiment, the cutter mechanism 312 separates the film 75 from the film roll FR. However, the present disclosure is not limited thereto. Instead of the cutter mechanism 312, the following configuration may be adopted. That is, lines of perforations (continuous small holes which transverse the film 75) are formed in the film 75 at regular intervals. After the film 75 is sandwiched by the film holder 331, the film holder 331 is rotated to apply horizontal force to the film 75, thereby cutting the film 75 along the perforations.

In the above embodiment, a specimen container 80 is held by the container holder 321. However, the present disclosure is not limited thereto. For example, when the sealing device 300 is configured as a single device, the user may manually hold a specimen container 80 to be sealed, instead of the container holder 321.

In the above embodiment, the film 75 is wound around a specimen container 80 by relatively rotating the film 75 and the specimen container 80 with the film 75 being in contact with the opening edge of the specimen container 80. However, the present disclosure is not limited thereto. As shown in FIG. 31, the film 75 may be adhered to the outer peripheral surface of the specimen container 80 without relatively rotating the film 75 and the specimen container 80. In the example of FIG. 31, the sealing device 300 is provided with an adhesion processing part 631 having a recess 632 into which the upper end portion of the specimen container 80 can be fitted. With the film 75 being in contact with the opening edge of the specimen container 80, the upper end portion of the specimen container 80 is fitted in the recess 632 of the adhesion processing part 631 together with the film 75 covering the opening 81. The film 75 is sandwiched between the opening edge (upper end surface) of the specimen container 80 and a bottom surface of the recess 632, and is adhered to the opening edge. A portion, of the film 75, on the outer peripheral side of the opening edge is sandwiched between the outer peripheral surface of the specimen container 80 and an inner peripheral surface of the recess 632, and is adhered to the outer peripheral surface of the specimen container 80. In this state, holding of the film 75 by the film holder 331 is released. The film 75 around the adhesion processing part 631 may be cut off. Thereafter, the specimen container 80 is moved to the outside of the recess 632 of the adhesion processing part 631, whereby sealing of the opening 81 is completed.

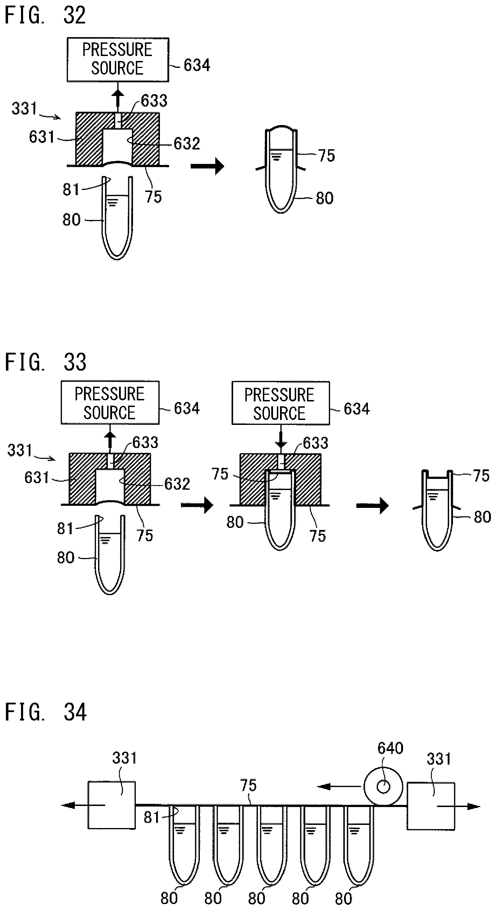

In the above embodiment, the film holder 331 sandwiches the film 75 by using the first member 331a and the second member 331b. However, the present disclosure is not limited thereto. As shown in FIG. 32, the film holder 331 may hold the film 75 by sucking the film 75. In the example of FIG. 32, the film holder 331 holds the film 75 by using the adhesion processing part 631. A negative pressure is supplied from a pressure source 634 through a pressure passage 633 formed at the bottom surface of the recess 632 of the adhesion processing part 631, whereby the film 75 is sucked and held on a lower surface of the adhesion processing part 631 (a lower opening of the recess 632). The method for sealing the opening 81 is similar to that shown in FIG. 31.

In the above embodiment, the protruding pressing part 335 disposed in the film holder 331 pushes the film 75 into an opening 81 of a specimen container 80. However, the present disclosure is not limited thereto. As shown in FIG. 33, the pressing part 335 may be configured to push the film 75 into the opening 81 by means of pressure. In the example of FIG. 33, the film holder 331 holds the film 75 by using the adhesion processing part 631. While the film 75 is held by means of negative pressure supplied into the recess 632 of the adhesion processing part 631, the upper end portion of the specimen container 80 is fitted into the recess 632 of the adhesion processing part 631 together with the film 75 covering the opening 81. While the upper end portion (opening edge) of the specimen container 80 is in contact with the bottom surface of the recess 632, the pressure source 634 supplies positive pressure through the pressure passage 633. A portion, of the film 75, covering the opening 81 is pushed into the opening 81 by the pressure supplied from the pressure source 634 to the bottom surface of the recess 632.

In the above embodiment, the specimen containers 80 are subjected to the sealing process one by one. However, the present disclosure is not limited thereto. The openings 81 of a plurality of specimen containers 80 may be collectively sealed through one sealing process. In the example of FIG. 34, while the opening edges of a plurality of (five in FIG. 34) specimen containers 80 are in contact with the lower surface of the film 75 held by the film holder 331, the upper surface of the film 75 is pressed against the opening edges of the specimen containers 80 by using a roller-like pressing member 640. The pressing member 640 is moved in an array direction of the plurality of specimen containers 80, whereby the film 75 is adhered to each of the opening edges of the respective specimen containers 80. In this state, holding of the film 75 by the film holder 331 is released. Thus, the openings 81 of the plurality of specimen containers 80 are collectively sealed with the film 75. Alternatively, the adhesion processing part 631 shown in any of FIGS. 31 to 33 may be provided with a plurality of recesses 632 so that a plurality of specimen containers 80 can be collectively sealed. Instead of the plurality of recesses 632, a groove-like recess 632 may be formed. In this case, a plurality of specimen containers 80 arrayed in a line may be fitted in the groove-like recess 632, and the plurality of specimen containers 80 may be collectively sealed.

In the above embodiment, the seal (second seal) is the film 75. However, the present disclosure is not limited thereto. The seal may have a cap-like structure in which, for example, an upper end surface of an annular peripheral wall member formed of a hard resin or the like is covered with a film or a thin member through which the aspiration tube 32 can penetrate.

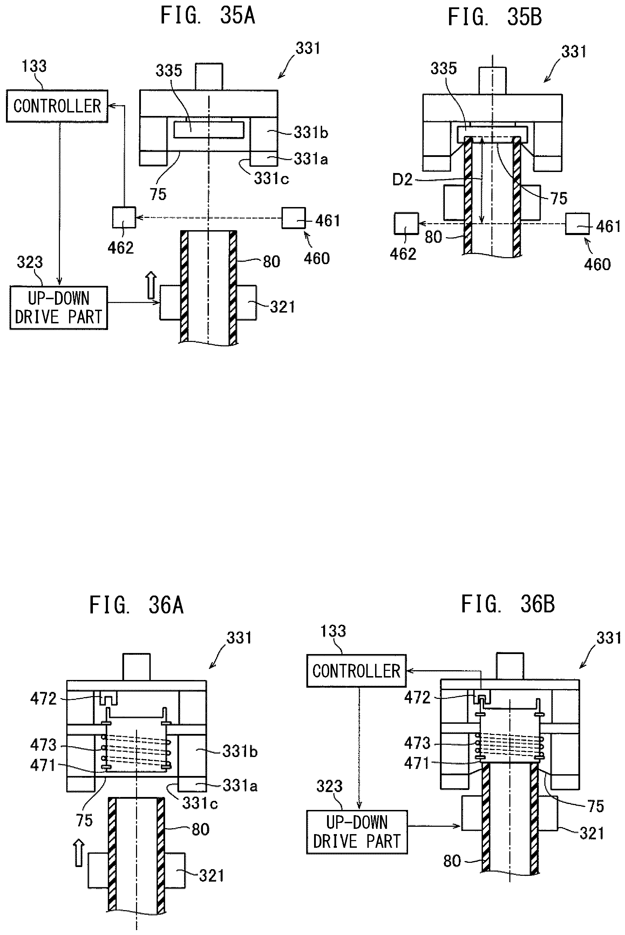

The specimen containers 80 shown in FIGS. 9 to 11, etc., have the same shape. However, the specimen containers 80 may have different lengths (heights). The sealing device 300 may be provided with a detector for detecting specimen containers 80 in order to accurately perform a sealing process on each of the specimen containers 80 having different lengths. For example, in the example of FIG. 35A, the sealing device 300 includes a detector 460. The detector 460 is a transmission type optical sensor, and includes a light emitter 461 and a light receiver 462. The light emitter 461 and the light receiver 462 are located at a height position between a specimen container 80 held in the specimen rack 85 and the film holder 331. The controller 133 causes the up-down drive part 323 of the container holding mechanism 320 to move the specimen container 80 upward, and receives a detection signal from the detector 460 when the upper end of the specimen container 80 is located at a detection position (on an optical path between the light emitter 461 and the light receiver 462, indicated by a broken line). Upon receiving the detection signal, the controller 133 controls the up-down drive part 323 so as to move the specimen container 80 upward from the detection position by a predetermined distance D2, as shown in FIG. 35B. When the specimen container 80 has been moved upward by the distance D2, the upper end of the specimen container 80 elastically deforms the pressing part 335 while stretching the film 75. With this configuration, the specimen container 80 can be moved upward with respect to the position of the upper end thereof, whereby the upper end of the specimen container 80 can be reliably located at an appropriate sealing position, regardless of the length of the specimen container 80.

In the example of FIG. 36A, a moving member 471 and a detector 472 are disposed inside the film holder 331. The moving member 471 is movable in the up-down direction when being pushed upward by the upper end of a specimen container 80. The detector 472 detects that the moving member 471 has reached a predetermined position. When the specimen container 80 held by the container holder 321 moves upward, the upper end of the specimen container 80 comes into contact with the film 75 and thereafter comes into contact with the moving member 471 via the film 75. As the specimen container 80 continues moving upward, the moving member 471 is moved upward while stretching the film 75. As shown in FIG. 36B, when the specimen container 80 reaches an appropriate position for the sealing process, the moving member 471 is detected by the detector 472. In FIGS. 36A and 36B, the detector 472 is a photo-interrupter, and detects a detection piece disposed on the moving member 471. Upon receiving the detection signal from the detector 472, the controller 133 controls the up-down drive part 323 so as to stop the upward movement of the specimen container 80. When the specimen container 80 is moved downward after the sealing process, the moving member 471 is returned to the initial position (see FIG. 36A) by an urging force of an urging member 473. This configuration also allows the upper end of the specimen container 80 to be reliably located at the sealing position, regardless of the length of the specimen container 80.

### (Configuration of specimen processing system)

In the above embodiment, the specimen processing system 100 including two specimen processing units, i.e., the specimen processing unit 140 and the specimen processing unit 150, has been described. However, the specimen processing system 100 may include one specimen processing unit as shown in FIGS. 22 to 24, 27 and the like, or may include three or more specimen processing units. For example, a specimen processing system may be provided with three specimen processing units including: a specimen processing unit for performing a urine qualitative test; a specimen processing unit for performing a urinary sediment test; and a specimen processing unit for capturing an image of cells in urine. A plurality of specimen processing devices may be included in one specimen processing unit. For example, one specimen processing unit may include: a specimen processing device 145 for performing a urine qualitative test; and a specimen processing device 155 for performing a urinary sediment test.

In the above embodiment, the transport device 20 (transport devices 111 to 171) transports the specimen rack 85 holding the specimen containers 80. However, the present disclosure is not limited thereto. The transport device 20 may directly transport the specimen containers 80 one by one without using the specimen rack 85.

In the above embodiment, the transport device 20 (transport devices 111 to 171) is configured to be able to transport the specimen rack 85 holding the specimen containers 80 from the loading unit 110 to the collection unit 170. However, the present disclosure is not limited thereto. In the present disclosure, the transport device 20 may be configured to be able to transport the specimen rack 85 between some of the units.

In the above embodiment, the specimen containers 80 are transported by the transport device 20. However, an operator may manually transport a specimen container 80 to the position of the sealing device 300 (sealing unit 10) or the position of the aspiration tube 32 of the specimen processing device. For example, in a case where a specimen processing unit and a sealing unit are not connected to each other but are installed as separate and independent devices, the operator may transport a specimen container 80 to a sealing device for a sealing process, and may transport the sealed specimen container 80 to the specimen processing unit for specimen aspiration and specimen processing.

In the above embodiment, the aspiration tube 32 of the specimen processing device penetrates through the seal (film 75). However, the present disclosure is not limited thereto. In the present disclosure, a puncture member for forming a through-hole TH may be provided separately from the aspiration tube 32, and this puncture member may penetrate through the seal (film).

### (Fourth specimen processing method)

That is, a fourth specimen processing method of the present embodiment is a method of processing a urine specimen 90 contained in a specimen container 80 having an opening 81, by using a specimen processing device. The method includes (1) a step of sealing, with a seal, the opening 81 of the specimen container 80 containing the urine specimen 90, (2) a step of forming, by using a puncture member of the specimen processing device, a through-hole TH in the seal with which the specimen container is sealed, (3) a step of aspirating, by using an aspiration tube 32, the urine specimen 90 in the specimen container 80 via the through-hole TH, and (4) a step of performing specimen processing on the aspirated urine specimen 90.

As the puncture member, a known needle member may be used. The puncture member forms the through-hole TH in a part of the seal, and the aspiration tube 32 is inserted into the specimen container 80 through the through-hole TH, whereby the urine specimen 90 is aspirated. The outer diameter (diameter) of the puncture member may be sufficiently smaller than the inner diameter of the opening 81 because the through-hole TH is only required to let the aspiration tube 32 pass through it.

Thus, the urine specimen 90 can be aspirated through the through-hole TH while the opening 81 of the specimen container 80 is sealed with the seal. Therefore, release of odor can be reduced when the urine specimen 90 is aspirated during the specimen processing. After aspiration of the urine specimen 90, the through-hole TH is left in a part of the seal. However, since the through-hole TH formed in the seal is sufficiently smaller than the opening 81 of the specimen container 80, release of odor can be effectively reduced as compared to the case where the opening 81 is fully opened.

### DESCRIPTION OF THE REFERENCE CHARACTERS

- 10, 130, 602, 614, 621: sealing unit
- 20, 111, 121, 131, 141, 151, 161, 171: transport device
- 30, 140, 150, 604, 615, 622: specimen processing unit
- 31, 145, 155: specimen processing device
- 32: aspiration tube
- 41, 331: film holder
- 42, 321: container holder
- 43, 323: up-down drive part
- 44, 333: rotation drive part
- 50, 120, 613: uncapping unit
- 71: seal
- 75: film (seal, second seal)
- 80: specimen container
- 81: opening
- 84: cap (first seal)
- 90: urine specimen
- 100: specimen processing system
- 114, 144, 154: information reader
- 133: controller
- 160, 623: second sealing unit
- 163: controller
- 300: sealing device
- 335: pressing part
- 341: film removal part
- 601: first loading unit
- 603: second loading unit
- 611: third loading unit
- 612: fourth loading unit

## Claims

1. A specimen processing method of processing, by using a specimen processing device, a urine specimen contained in a specimen container having an opening, the method comprising:
sealing, with a seal, the opening of the specimen container containing the urine specimen;
aspirating the urine specimen in the specimen container by using an aspiration tube, of the specimen processing device, which has penetrated through the seal; and
processing the aspirated urine specimen.

2. The specimen processing method of claim 1, further comprising
transporting the specimen container sealed with the seal to an aspiration position of the aspiration tube.

3. The specimen processing method of claim 1 or 2, further comprising
removing a cap from the specimen container having the cap attached to the opening, wherein
the sealing with the seal is performed on the specimen container from which the cap is removed.

4. The specimen processing method of any one of claims 1 to 3, wherein the seal is a film.

5. The specimen processing method of claim 4, wherein the film is a resin film having extensibility.

6. The specimen processing method of any one of claims 1 to 5, further comprising
re-sealing the opening of specimen container, from which the urine specimen has been aspirated by the aspiration tube penetrating the seal, with a seal through which the aspiration tube can penetrate.

7. The specimen processing method of claim 6, further comprising
acquiring identification information for identifying the specimen container, wherein
the re-sealing of the specimen container is selectively performed on the basis of the identification information.

8. The specimen processing method of any one of claims 1 to 7, wherein
in the sealing with the seal, a sealing method to be performed is selected from among a plurality of sealing methods.

9. The specimen processing method of any of claims 1 to 8, wherein
the sealing with the seal includes:
stretching the seal by pressing an opening edge of the specimen container against the seal; and
winding the seal around the specimen container by rotating at least one of the seal in a stretched state and the specimen container around a center axis of the specimen container.

10. The specimen processing method of claim 9, wherein
the winding of the seal around the specimen container is performed by rotating the seal in the stretched state around the center axis of the specimen container.

11. A specimen processing system comprising:
a sealing device configured to seal, with a seal, an opening of a specimen container containing a urine specimen;
a transport device configured to transport the sealed specimen container; and
a specimen processing device including an aspiration tube configured to penetrate through the seal and aspirate the urine specimen from the specimen container, the specimen processing device being configured to process the aspirated urine specimen.

12. The specimen processing system of claim 11 further comprising
an uncapping device configured to remove a cap from the specimen container having the cap attached to the opening, wherein
the sealing device seals, with the seal, the specimen container from which the cap is removed.

13. The specimen processing system of claim 11 or 12, wherein the seal is a film.

14. The specimen processing system of claim 13, wherein the film is a resin film having extensibility.

15. The specimen processing system of any one of claims 11 to 14, further comprising
a second sealing device configured to re-seal the opening of the specimen container, from which the urine specimen has been aspirated, with a seal through which the aspiration tube can penetrate.

16. The specimen processing system of claim 15, including a plurality of the specimen processing devices and a plurality of the second sealing devices, wherein
the plurality of second sealing devices perform re-sealing on specimen containers that have been subjected to aspiration in the specimen processing devices that are different from each other.

17. The specimen processing system of claim 15 or 16, further comprising:
an information reader configured to read identification information for identifying the specimen container; and
a controller configured to control the second sealing device so as to selectively perform re-sealing on the specimen container on the basis of the read identification information.

18. The specimen processing system of any one of claims 11 to 17, further comprising
a controller configured to perform control of selecting a sealing method to be performed by the sealing device from among a plurality of sealing methods.

19. A specimen processing method of processing, by using a specimen processing device, a urine specimen contained in a specimen container having an opening, the method comprising:
aspirating the urine specimen from the specimen container containing the urine specimen, by using an aspiration tube of the specimen processing device;
processing the aspirated urine specimen; and
sealing, with a seal, the opening of the specimen container from which the urine specimen has been aspirated.

20. The specimen processing method of claim 19, wherein the seal is a film.

21. The specimen processing method of claim 20, wherein the film is a resin film having extensibility.

22. The specimen processing method of claim 20 or 21, wherein
the sealing with the seal includes:
stretching the film by pressing an opening edge of the specimen container against the film; and
winding the film around the specimen container by rotating at least one of the film in a stretched state and the specimen container around a center axis of the specimen container.

23. The specimen processing method of claim 22, wherein
the winding of the seal around the specimen container is performed by rotating the seal in the stretched state around the center axis of the specimen container.

24. A sealing device configured to seal an opening of a specimen container having the opening, comprising:
a film holder configured to hold a film;
a container holder configured to hold the specimen container in which a specimen is contained;
an up-down drive part configured to drive at least one of the film holder and the container holder in an up-down direction;
a rotation drive part configured to drive at least one of the film holder and the container holder so as to rotate around a center axis of the specimen container; and
a controller configured to perform control of winding the film around the specimen container by operating the rotation drive part in a state where the film held by the film holder is, by the up-down drive part, pressed against an opening edge of the specimen container held by the container holder.

25. The sealing device of claim 24, wherein
the controller controls the rotation drive part to operate until the film that is wound around the specimen container is separated from a portion, of the film, held by the film holder.

26. The sealing device of claim 24 or 25, further comprising
a film removal part configured to remove a portion, of the film, which is held by the film holder, from the film holder.

27. The sealing device of any one of claims 24 to 26, further comprising
a pressing part configured to push the film covering the opening of the specimen container into the opening.

28. The sealing device of any one of claims 24 to 27, wherein the film is a resin film having extensibility.

29. A specimen processing method of processing, by using a specimen processing device, a urine specimen contained in a specimen container having an opening, the method comprising:
sealing, with a seal, the opening of the specimen container containing the urine specimen;
forming, by a puncture member of the specimen processing device, a through-hole in the seal with which the specimen container is sealed;
aspirating the urine specimen in the specimen container through the through-hole by using an aspiration tube; and
processing the aspirated urine specimen.
